(19) **Europäisches Patentamt / European Patent Office / Office européen des brevets**

(11) **EP 4 733 387 A1**

(12) # EUROPEAN PATENT APPLICATION
published in accordance with Art. 153(4) EPC

(43) Date of publication:
**29.04.2026 Bulletin 2026/18**

(21) Application number: **24825328.8**

(22) Date of filing: **21.06.2024**

(51) International Patent Classification (IPC):
**C12N 1/20** (2026.01)   **A61K 35/747** (2015.01)
**A61P 1/10** (2006.01)

(52) Cooperative Patent Classification (CPC):
**A61K 35/747; A61P 1/10; C12N 1/20**

(86) International application number:
**PCT/CN2024/100577**

(87) International publication number:
**WO 2024/260441 (26.12.2024 Gazette 2024/52)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **21.06.2023   CN 202310744483**
**28.09.2023   CN 202311294650**
**28.09.2023   CN 202311294783**

(71) Applicant: **COREE Company Limited**
**Hong Kong 999077 (HK)**

(72) Inventors:
- **WANG, Tingting**
  **Beijing 101300 (CN)**
- **ZHANG, Shiqi**
  **Beijing 101300 (CN)**

- **FAN, Linlin**
  **Beijing 101300 (CN)**
- **ZHAO, Ying**
  **Beijing 101300 (CN)**
- **DING, Yan**
  **Beijing 101300 (CN)**
- **ZHANG, Di**
  **Beijing 101300 (CN)**
- **LEE, Soowon**
  **Beijing 101300 (CN)**
- **LIM, Chongyoon**
  **Beijing 101300 (CN)**

(74) Representative: **Studio Torta S.p.A.**
**Via Viotti, 9**
**10121 Torino (IT)**

Remarks:
The complete document including Reference Table(s) and the Sequence Listing(s) can be downloaded from the EPO website

(54) **COMPOUND BACTERIAL POWDER PREPARATION, AND PREPARATION METHOD THEREFOR AND USE THEREOF**

(57)   The present invention relates to the technical field of microorganisms, in particular to a compound bacterial powder preparation, preparation method and use thereof. The compound bacterial powder preparation comprises a *Bifidobacterium animalis* (*B. animalis*) strain, a *Lactiplantibacillus plantarum* (*L. plantarum*) strain and a *Bacillus coagulans* (*B. coagulans*), wherein the deposit number of the *B. animalis* strain is CGMCC No. 25681. The compound bacterial powder preparation can stimulate mouse macrophages RAW264.7 to produce cytokines TNF-α and IL-6, thereby enhancing immunity; can significantly enhance mouse cellular immune function, humoral immune function, and monocyte-macrophage function, thus having the function of enhancing immunity; and can increase the ink propulsion rate, and has the potential functional of defecation.

FIG. 10

**Description**

**CROSS-REFERENCE TO RELATED APPLICATIONS**

This application claims priority to Chinese Patent Application No. 202310744483.5 filed in China on June 21, 2023, Chinese Patent Application No. 202311294783.4 filed in China on September 28, 2023, and Chinese Patent Application No. 202311294650.7 filed in China on September 28, 2023, the entire contents of which are incorporated herein by reference.

**TECHNICAL FIELD**

The present invention relates to the technical field of microorganisms, in particular to a compound bacterial powder preparation, preparation method and use thereof.

**BACKGROUND**

Bifidobacterium is an important component of human intestinal flora, the number of which is closely related to human health conditions, and has many important physiological functions to human health, such as biological barrier, nutritional effect, anti-tumor effect, improvement of gastrointestinal function and anti-aging.

*Bifidobacterium animalis* (*B. animalis*) is divided into two subspecies, which are *B. animalis* subsp. *animalis* and *B. animalis* subsp. *lactis,* which have the function of maintaining intestinal microecological balance and improving immunity, and have good safety.

The compound bacterial powder preparation is a preparation obtained by mixing two or more probiotics in a certain ratio, and each strain in the preparation synergizes to improve specific functions.

Currently, the research on the compound bacterial powder preparation between *B. animalis* and other probiotics is still insufficient. Therefore, it is crucial to provide a compound bacterial powder preparation of a *B. animalis* with excellent performance.

**SUMMARY**

Objective of the Invention

An object of the present invention is to provide a compound bacterial powder preparation, which can stimulate mouse macrophages RAW264.7 to produce cytokines TNF-$\alpha$ and IL-6, improve immunity and maintain immune balance.

Another object of the present invention is to provide a compound bacterial powder preparation, which can significantly enhance mouse cellular immune function, humoral immune function, and monocyte-macrophage function, thus having the function of enhancing immunity; and increases the ink propulsion rate, thus having the potential of defecation function.

Technical Solution

In order to achieve the above objects, the present invention provides the following technical solutions:

In one aspect, the present invention provides a compound bacterial powder preparation, comprising a *B. animalis* strain, a *Lactiplantibacillus plantarum* (*L. plantarum*) strain and a *Bacillus coagulans* (*B. coagulans*) strain, wherein the deposit number of the *B. animalis* strain is CGMCC No. 25681.

In one embodiment, the compound bacterial powder preparation consists of *B. animalis* HOM2120 strain, *L. plantarum* HOM2217 strain and *B. coagulans* HOM5301 strain.

In one embodiment, the *B. animalis* strain comprises a sequence of a 16S rRNA gene represented by SEQ ID NO: 1.

In another embodiment, the *L. plantarum* strain comprises a sequence of a 16S rRNA gene represented by SEQ ID NO: 12.

Preferably, the ratio of the *B. animalis* strain, the *L. plantarum* strain and the *B. coagulans* strain is 1-3 : 1-3 : 1-3, based on the number of viable bacteria.

More preferably, the ratio of the *B. animalis* strain, the *L. plantarum* strain and the *B. coagulans* strain is 1:1:1, based on the number of viable bacteria.

In another embodiment, the deposit number of the *L. plantarum* strain is CGMCC No. 25683.

In another aspect, the present invention provides the compound bacterial powder preparation described above for use as a medicament for defecation.

The present invention also provides the compound bacterial powder preparation described above for use in producing extracellular polysaccharides, lactic acid and short-chain fatty acids.

The present invention also provides the compound bacterial powder preparation described above for use as a medicament for improving immunity and maintaining immune balance.

Preferably, the compound bacterial powder preparation is used to produce cytokine, which is tumor necrosis factor-$\alpha$ (TNF-$\alpha$) and interleukin-6 (IL-6).

In yet another aspect, the present invention provides a method for preparing the compound bacterial powder preparation as described above, comprising: mixing a *B. animalis* strain, a *L. plantarum* strain and a *B. coagulans* strain, and compounding.

Preferably, the ratio of the *B. animalis* strain, the *L. plantarum* strain and the *B. coagulans* strain is 1-3 : 1-3 : 1-3, based on the number of viable bacteria.

More preferably, the ratio of the *B. animalis* strain, the *L. plantarum* strain and the *B. coagulans* strain is 1:1:1, based on the number of viable bacteria.

In one embodiment, the compound bacterial powder preparation of the present invention may be in the form of solution, powder, granules, tablet, or capsule.

In another embodiment, the *L. plantarum* strain used in the present invention is isolated from healthy human breast milk, and the *B. animalis* is isolated from the feces of healthy infants.

Advantageous Effects

The compound bacterial powder of the present invention has the following beneficial functions:

1. The compound bacterial powder preparation of the present invention can stimulate mouse macrophages RAW264.7 to produce cytokines TNF-$\alpha$ and IL-6, thereby enhancing immunity.
2. The compound bacterial powder preparation of the present invention can significantly enhance mouse cellular immune function, humoral immune function, and monocyte-macrophage function, thus having the function of enhancing immunity; and can increase the ink propulsion rate, and has the potential functional of defecation.
3. The compound bacterial powder preparation of the present invention can produce high levels of extracellular polysaccharides, lactic acid and short-chain fatty acids; can inhibit various pathogenic bacteria; and can adhere to intestinal epithelial cells.

In addition, the compound bacterial powder preparation of the present invention also has the function of losing weight, which can inhibit the growth of the obesity-related strain *Enterobacter cloacae*, inhibit the differentiation of 3T3-L1 precursor adipocytes, thereby achieving the effect of effectively inhibiting fat deposition. It can reduce the content of cholesterol; and can reduce the body weight of obese model rats, reduce the total weight gain and total food utilization rate of obese model rats, significantly reduce the body fat weight and fat/body weight ratio of obese model rats, increase the content of serum high-density lipoprotein cholesterol, effectively increase the content of intestinal short-chain fatty acids, and reduce the expression of cellular inflammatory factor TNF-$\alpha$ in serum. It can also significantly reduce the body weight of obese model mice, and reduce the total weight gain and total food utilization rate of obese model rats.

**BRIEF DESCRIPTION OF THE DRAWINGS**

FIG. 1A shows a phylogenetic tree of *B. animalis* subsp. *lactis* HOM2120 strain used in the present invention; FIG. 1B shows a phylogenetic tree of *L. plantarum* HOM2217 used in the present invention.

FIG. 2 shows the RAPD cluster analysis diagram of *B. animalis* subsp. *lactis* HOM2120 strain of the present invention constructed based on the UPGMA method.

FIG. 3 shows the effect of *B. animalis* subsp. *Lactis* HOM2120 strain of the present invention on the cell viability of mouse macrophages RAW264.7.

FIG. 4 shows the effect of *B. animalis* subsp. *lactis* HOM2120 strain of the present invention on the IL-6 secretion of mouse macrophage.

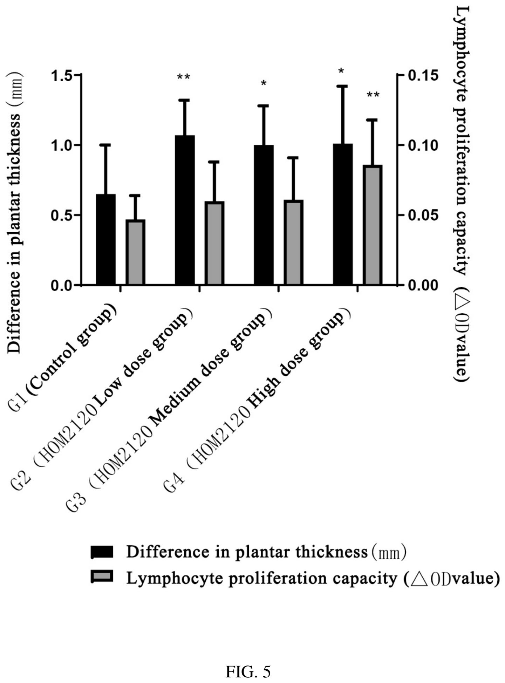

FIG. 5 shows the enhancement of cellular immune function by *B. animalis* subsp. *lactis* HOM2120 of the present invention.

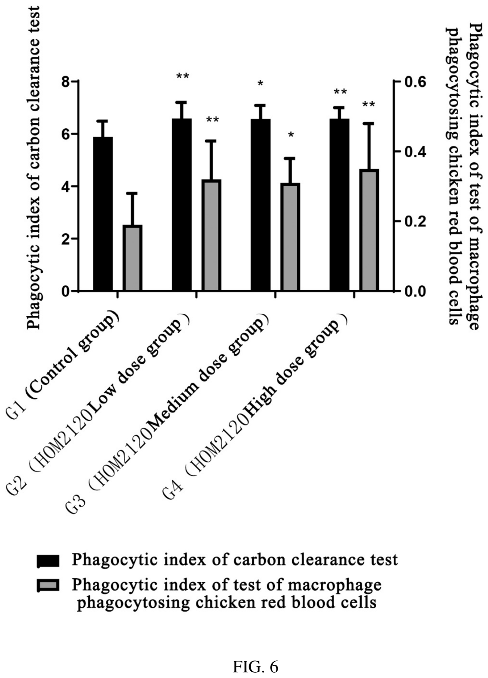

FIG. 6 shows the enhancement of monocyte-macrophage function by *B. animalis* subsp. *lactis* HOM2120 of the present invention.

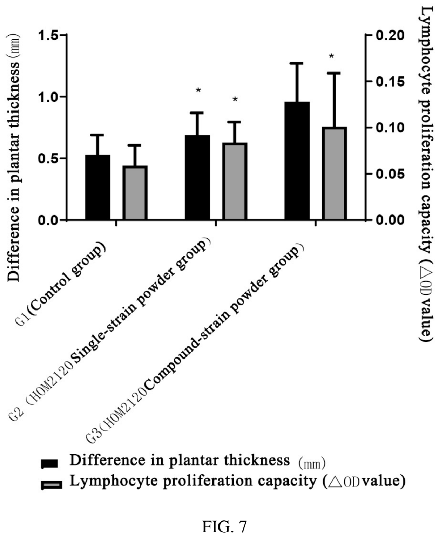

FIG. 7 shows the enhancement of cellular immune function by single-strain powder and compound bacterial powder of *B. animalis* subsp. *lactis* HOM2120 of the present invention. * indicates $p < 0.05$ compared to the control group, **

indicates p < 0.01 compared to the control group, *** indicates p < 0.001 compared to the control group.

FIG. 8 shows the enhancement of humoral immune function by the compound bacterial powder of *B. animalis* subsp. *lactis* HOM2120 of the present invention. * indicates p < 0.05 compared to the control group, ** indicates p < 0.01 compared to the control group, *** indicates p < 0.001 compared to the control group.

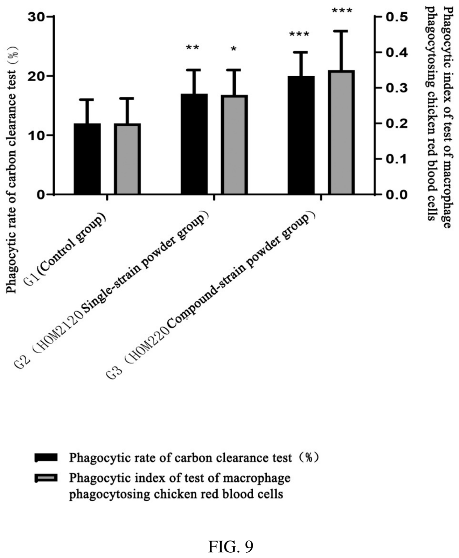

FIG. 9 shows the enhancement of monocyte-macrophage function by single-strain powder and compound bacterial powder of *B. animalis* subsp. *lactis* HOM2120 of the present invention. * indicates p < 0.05 compared to the control group, ** indicates p < 0.01 compared to the control group, *** indicates p < 0.001 compared to the control group.

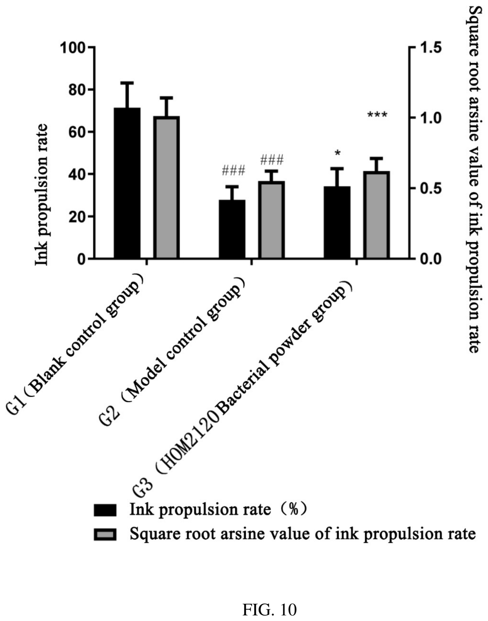

FIG. 10 shows the defecation function of the *B. animalis* subsp. *lactis* HOM2120 single-strain powder of the present invention.

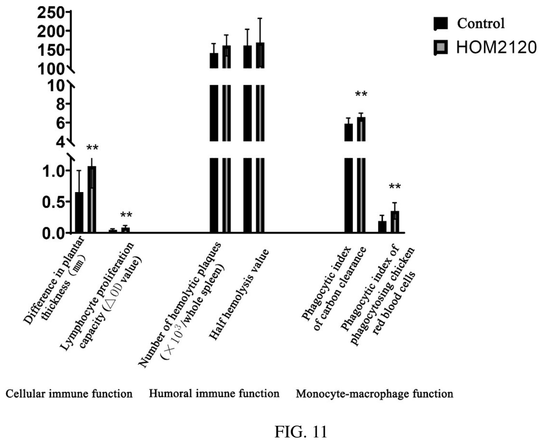

FIG. 11 shows the results of *B. animalis* subsp. *lactis* HOM2120 of the present invention enhancing the mouse cellular immune function, humoral immune function, and monocyte-macrophage function.

**Microbiological Collection**

**1. *Bifidobacterium animalis* (*B. animalis*) HOM2120 strain of the present invention**

The B. animalis HOM2120 strain of the present invention was deposited in the China General Microbiological Culture Collection Center (CGMCC) on September 9, 2022, with a deposit address is: Institute of Microbiology, Chinese Academy of Sciences, No. 3, Courtyard 1, Beichen West Road, Chaoyang District, Beijing; and a deposit number of CGMCC No. 25681.

The *B. animalis* HOM2120 strain of the present invention was sent to the Institute of Microbiology, Chinese Academy of Sciences for identification in May 2023.

The detection and identification conclusions are as follows: Under the conditions of this laboratory, according to the comprehensive analysis of experimental data such as cell morphology, physiological and biochemical characteristics, 16S rRNA gene sequence, *tuf* gene sequence of the test strain, referring to the "*Bergey's Manual of Systematic Bacteriology*" and relevant research paper of International Journal of Systematic and Evolutionary Microbiology, the identification result of the test strain (strain number: HOM2120) is: *B. animalis* subsp. *lactis.*

The cell morphology of this strain is: polymorphic rod-shaped; the physiological and biochemical characteristics are: Gram-positive, catalase negative (-), oxidase negative (-); 16S rRNA gene sequence is shown in SEQ ID NO: 1, and *tuf* gene sequence is shown in SEQ ID NO: 9.

**2. *L. plantarum* HOM2217 strain**

The *Lactiplantibacillus plantarum* (*L. plantarum*) HOM2217 strain of the present invention, also known as *Lactobacillus plantarum* HOM2217 strain, was deposited in the China General Microbiological Culture Collection Center (CGMCC) on September 9, 2022, with deposit number of CGMCC No. 25683; the deposit address is: Institute of Microbiology, Chinese Academy of Sciences, No. 3, Courtyard 1, Beichen West Road, Chaoyang District, Beijing.

**Identification**

The *L. plantarum* HOM2217 strain of the present invention, also known as *Lactobacillus plantarum* HOM2217 strain, was sent to the Institute of Microbiology, Chinese Academy of Sciences for identification in May 2023.

The detection and identification conclusions are as follows: Under the conditions of this laboratory, according to the comprehensive analysis of experimental data such as cell morphology, physiological and biochemical characteristics, 16S rRNA gene sequence, *pheS* gene sequence of the test strain, and referring to the "Bergey's Manual of Systematic Bacteriology" and relevant research paper of International Journal of Systematic and Evolutionary Microbiology, the identification result of the test strain (strain number: HOM2217) is: *Lactiplantibacillus plantarum.* Synonym: *Lactobacillus plantarum.*

The cell morphology of this strain is: rod-shaped; the physiological and biochemical characteristics are: Gram-positive, catalase negative (-), oxidase negative (-); 16S rRNA gene sequence is shown in SEQ ID NO: 12, and *pheS* gene sequence is shown in SEQ ID NO: 13.

**DETAILED DESCRIPTION**

The present invention discloses strains, characteristics and applications, and those skilled in the art can appropriately improve process parameters with reference to the content herein. It should be particularly noted that all similar substitutions and amendments are obvious to those skilled in the art, and are considered to be included in the present invention. The method and application of the present invention have been described through the preferred embodiments. It is obvious that those skilled in the art can make amendments or appropriate changes and combinations to the method and application described herein without departing from the content, spirit and scope of the present invention to implement and apply the technology of the present invention.

In order to further illustrate the technical means and effects adopted by the present invention to achieve the intended purpose, the technical solution of the present invention will be further described below in conjunction with specific examples, but are not limited thereto. Any amendments or equivalent substitutions to the technical solutions of the present invention, without departing from the spirit and scope of the technical solutions of the present invention, shall fall within the protection scope of the present invention. The experimental methods without specific conditions in the following embodiments are implemented according to conventional methods and conditions in the art.

As identified above, the *Bifidobacterium animalis* (*B. animalis*) HOM2120 strain in the present invention is identified as *B. animalis* subsp. *lactis*. Therefore, the *B. animalis* HOM2120 strain used in the present invention refers to the *B. animalis* subsp. *lactis* HOM2120 strain.

In the present invention, "*Lactiplantibacillus plantarum*" and "*Lactobacillus plantarum*" are used interchangeably.

In the present invention, *Bacillus coagulans* (*B. coagulans*) and *Weizmannia coagulans* are used interchangeably.

The detailed information of the *B. coagulans* HOM5301 strain used in the compound bacterial powder of the present invention is described in the authorized Chinese patent CN112522134B, whose deposit number is CGMCC No. 20383. The contents of CN112522134B are incorporated herein by reference.

In order to make the technical problems to be solved of the present invention, technical solutions adopted and advantages clearer, the present invention will be described in detail below with reference to the drawings and specific embodiments. The following examples are intended to illustrate the present invention, but are not intended to limit the scope of the present invention.

It should be noted that the experimental methods used in the present invention are conventional methods unless otherwise specified.

Unless otherwise specified, the reagents and materials used in the present invention are all prepared by conventional methods or obtained commercially.

The *B. animalis* subsp. *lactis* used in the present invention was isolated from feces of healthy infants, and the specific isolation method is as described in Example 1.

**Example**

**Example 1-1. Isolation and Identification of *B. animalis* subsp. *lactis* HOM2120**

(1) Preparation of Artificial Gastrointestinal Fluid

Artificial gastric fluid: Into 16.4 mL of dilute hydrochloric acid (1 mol/L), 800 mL of water was added, the pH was adjusted to 3.0, and 10g of pepsin was added. The mixture was shaken well, added with water to 1000 mL, centrifuged at 5000 rpm for 5 min, the supernatant was taken, filtered with a 0.22$\mu$m filter membrane for sterilization, and stored at -20°C for later use.

Artificial intestinal fluid: 6.8g of potassium dihydrogen phosphate was weighted, 500 mL of water was added, and the pH was adjusted to 6.8 with a 0.4% (0.1 mol/L) sodium hydroxide solution. Separately, 10 g of pancreatin and 3 g of pig bile salt were weighted, an appropriate amount of water was added to dissolve them. The two solutions were mixed, added with water to 1000 mL, centrifuged at 5000 rpm for 5 min. The supernatant was taken, filtered and sterilized with a 0.22 $\mu$m filter membrane, and stored at -20°C for later use.

Preparation of Isolation Medium

MRS-Cys liquid medium: 0.5 g of L-cysteine hydrochloride was added to each liter of MRS broth medium (Cat. No. CM1163, OXOID, UK), sterilized at 121°C for 15 min, and stored at 2 ~ 8°C for one week in the dark.

MRS-Cys solid medium containing bromocresol purple: 0.04g of bromocresol purple was added to each liter of MRS solid medium (Cat. No.: CM1175, OXOID, UK), and stirred well. Sterilization was performed at 121°C for 15 min, about 15 mL of the medium was poured into each petri dish in a clean bench and was allowed to solidify for later use.

(3) Isolation and Screening of *B. animalis* subsp. *lactis* HOM2120

The *B. animalis* subsp. *lactis* of the present invention is isolated from feces of healthy infants.

The extracorporeal feces were collected with a sterile anaerobic tube and stored at low temperature under anaerobic environment. The experiment began on the day of sampling. About 1 g of fecal sample was weighted and added into an anaerobic tube containing 9 mL of MRS-Cys liquid medium, anaerobically incubated at 37°C for 24 h. After centrifugation at 8000 rpm for 10 min, the supernatant was discarded, and 10 mL of artificial gastric fluid was added. After mixing, the mixture was incubated anaerobically at 37°C for 3 h, centrifuged at 8000 rpm for 10 min, the supernatant was discarded. 10 mL of artificial intestinal fluid was added, after mixing, the mixture was incubated anaerobically at 37°C for 3 h. The sample was diluted by a 10-fold dilution method, diluted to a concentration of $10^{-6}$. 100µL of each dilution was taken from the original solution to the concentration of $10^{-6}$, and spread onto MRS-Cys solid medium plates containing bromocresol purple, anaerobically static incubated at 37°C for 72 h.

Single colonies with yellowing edges were picked, streaked, and purified 3-4 times until single colonies were observed. Gram staining and microscopy of colony morphology were performed simultaneously. Single colonies were then transferred to liquid medium for pure culture, preserved with glycerol, and stored at -80° C.

(4) Morphological Observation of *B. animalis* subsp. *lactis* HOM2120 Strain

*B. animalis* subsp. *lactis* HOM2120 was anaerobically cultured on MRS agar medium at 37°C for 48 h. The colonies were round, milky white, the surface was moist and smooth, the center was raised, and the edges were neat. The diameter of the colonies was about 2.5 mm. It was observed under an optical microscope that the bacteria were rod-shaped with enlarged ends, club-shaped or Y-shaped bacteria. The size of the bacteria was about $(0.5 \sim 0.9)$ µm $\times (3 \sim 6)$ µm, arranged individually or in pairs, Gram-positive, and did not form spores. At the same time, the other two strains with the same morphology were screened, and named BH13-25 and BH13-33.

(5) Identification of *B. animalis* subsp. *lactis* HOM2120 Strain

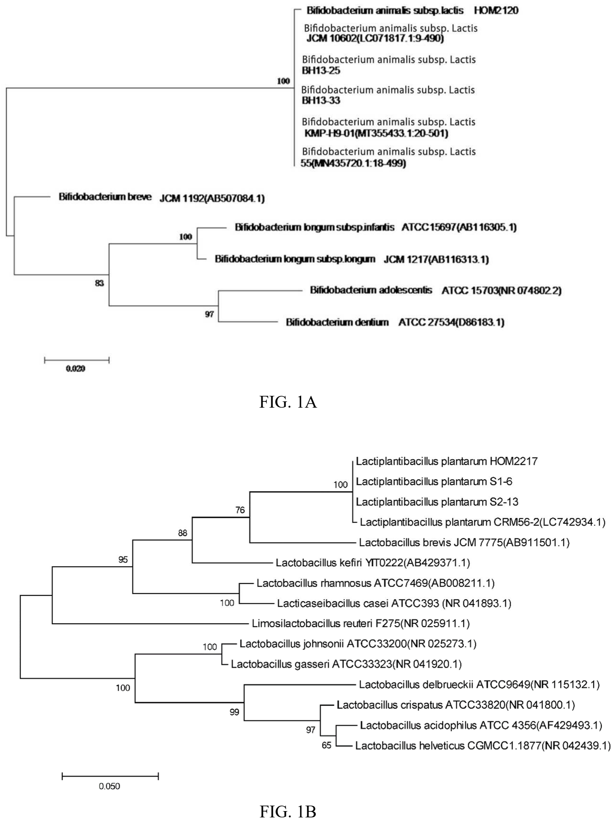

16S rRNA gene identification of strains: DNA was extracted from the stored three strains HOM2120, BH13-25 and BH13-33, respectively, and 16S rRNA gene amplification was performed. PCR amplification and agarose gel electrophoresis were performed using universal primers 27F: SEQ ID NO: 7; 1492 R: SEQ ID NO: 8 (see Table 1). Then, the gel was cut and recovered, sequenced, and the isolated strain was sequenced for 16S rRNA gene. According to its 16S rRNA gene sequence, it was aligned in the NCBI database using the BLAST tool, and the phylogenetic tree was constructed using Mega 7.0 software, as shown in FIG. 1A. The identification results of the three strains were all *B. animalis* subsp.*lactis,* which were named *B. animalis* subsp.*lactis* HOM2120, BH13-25 and BH13-33 respectively, wherein the sequence of the 16S rRNA gene of HOM2120 was shown in SEQ ID NO: 1:

TGCAGTCGAACGGGATCCCTGGCAGCTTGCTGTCGGGGTGAG

AGTGGCGAACGGGTGAGTAATGCGTGACCAACCTGCCCTGTGCACCGGAA

TAGCTCCTGGAAACGGGTGGTAATACCGGATGCTCCGCTCCATCGCATGGT

GGGGTGGGAAATGCTTTTGCGGCATGGGATGGGGTCGCGTCCTATCAGCTT

GTTGGCGGGGTGATGGCCCACCAAGGCGTTGACGGGTAGCCGGCCTGAGA

GGGTGACCGGCCACATTGGGACTGAGATACGGCCCAGACTCCTACGGGAG

GCAGCAGTGGGGAATATTGCACAATGGGCGCAAGCCTGATGCAGCGACGC

CGCGTGCGGGATGGAGGCCTTCGGGTTGTAAACCGCTTTTGTTCAAGGGCA

AGGCACGGTTTCGGCCGTGTTGAGTGGATTGTTCGAATAAGCACCGGCTAA

CTACGTGCCAGCAGCCGCGGTAATACGTAGGGTGCGAGCGTTATCCGGATT

TATTGGGCGTAAAGGGCTCGTAGGCGGTTCGTCGCGTCCGGTGTGAAAGTC

CATCGCCTAACGGTGGATCTGCGCCGGGTACGGGCGGGCTGGAGTGCGGT

AGGGGAGACTGGAATTCCCGGTGTAACGGTGGAATGTGTAGATATCGGGAA

GAACACCAATGGCGAAGGCAGGTCTCTGGGCCGTCACTGACGCTGAGGAG

CGAAAGCGTGGGGAGCGAACAGGATTAGATACCCTGGTAGTCCACGCCGT

AAACGGTGGATGCTGGATGTGGGGCCCTTTCCACGGGTCCCGTGTCGGAG

CCAACGCGTTAAGCATCCCGCCTGGGGAGTACGGCCGCAAGGCTAAAACT

CAAAGAAATTGACGGGGGCCCGCACAAGCGGCGGAGCATGCGGATTAATT

CGATGCAACGCGAAGAACCTTACCTGGGCTTGACATGTGCCGGATCGCCGT

GGAGACACGGTTTCCCTTCGGGGCCGGTTCACAGGTGGTGCATGGTCGTC

GTCAGCTCGTGTCGTGAGATGTTGGGTTAAGTCCCGCAACGAGCGCAACC

CTCGCCGCATGTTGCCAGCGGGTGATGCCGGGAACTCATGTGGGACCGCCG

GGGTCAACTCGGAGGAAGGTGGGGATGACGTCAGATCATCATGCCCCTTAC

GTCCAGGGCTTCACGCATGCTACAATGGCCGGTACAACGCGGTGCGACAC

GGTGACGTGGGGCGGATCGCTGAAAACCGGTCTCAGTTCGGATCGCAGTC

TGCAACTCGACTGCGTGAAGGCGGAGTCGCTAGTAATCGCGGATCAGCAA

CGCCGCGGTGAATGCGTTCCCGGGCCTTGTACACACCGCCCGTCAAGTCAT

GAAAGTGGGTAGCACCCGAAGCCGGTGGCCCGACCCTTGTGGGGGGA

In addition, *B. animalis* subsp. *lactis* BH13-25 and BH13-33, isolated simultaneously with *B. animalis* subsp. *lactis* HOM2120 of the present invention, were used as control strains in the present invention, and their 16S rRNA genes sequences were shown in SEQ ID NOs: 10 and 11, respectively. By comparing the 16S rRNA gene sequences of the *B. animalis* subsp. *lactis* HOM2120 strain of the present invention and the *B. animalis* subsp. *lactis* BH13-25 and BH13-33 strains used as control strains in the present invention, it can be seen that the strains BH13-25 and BH13-33 have 1396 bases the same as the HOM2120 strain of the present invention, with a sequence similarity is 99.94%.

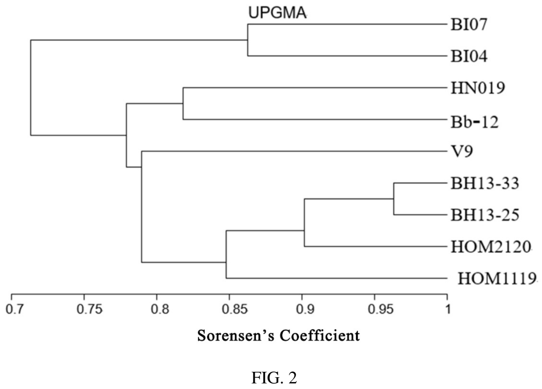

(6) Identification of strain by random amplification polymorphism DNA (RAPD): DNA was extracted from the stored strain, using the DNA of the strain as a template, and 5 primers OPA-02: SEQ ID NO: 2; OPA-18: SEQ ID NO: 3; OPL-07: SEQ ID NO: 4; OPL-16: SEQ ID NO: 5; OPM-05: SEQ ID NO: 6 were used to amplify DNA fragments capable of showing polymorphism by PCR, different DNA differences were present after gel electrophoresis, and the results were analyzed by cluster analysis software, as shown in FIG. 2. As shown in FIG. 2, *B. animalis* subsp. *lactis* HOM2120 was different from some commercial *B. animalis* subsp. *Lactis* strain and was unique.

Table 1

| Primers | Sequence | SEQ ID NO |
|---------|----------|-----------|
| OPA-02 | 5'- TGC CGA GCT G -3' | 2 |
| OPA-18 | 5'- AGG TGA CCG T -3' | 3 |
| OPL-07 | 5'- AGG CGG GAA C -3' | 4 |
| OPL-16 | 5'- AGG TTG CAG G -3' | 5 |
| OPM-05 | 5'- GGG AAC GTG T -3' | 6 |
| 27F | 5'- AGTTTGATCMTGGCTCAG -3' | 7 |
| 1492R | 5'- GGTTACCTTGTTACGACTT -3' | 8 |

(7) Genome resequencing: DNA was extracted from the stored strain and randomly fragmented. The DNA fragments of a required length were recovered by electrophoresis, and adapters were added for cluster preparation. Sequencing was performed using the Illumina platform. Through data analysis, assembly, and determination, the GC content was found to be 60.44%. By comparing genomics methods, the whole genome obtained by *B. animalis* subsp. *lactis* was resequenced and compared with the reference genome of *B. animalis* subsp. *lactis* published on the NCBI website for single nucleotide polymorphism (SNP) analysis. The results were shown in Table 2.

Table 2. SNP analysis results of *B. animalis* subsp. *lactis* HOM2120

| Strains | SNP Differences |
|---------|-----------------|
| *B. animalis* subsp. *lactis* AD011 | 188 |
| *B. animalis* subsp. *lactis* Bi-07 | 37 |
| *B. animalis* subsp. *lactis* HOM1119 | 74 |
| *B. animalis* subsp. *lactis* DSM.10140 | 35 |
| *B. animalis* subsp. *lactis* HN019 | 40 |
| *B. animalis* subsp. *animalis* IM386 | 60803 |
| *B. animalis* subsp. *lactis* V9 | 34 |
| *B. animalis* subsp. *lactis* BH13-33 | 190 |
| *B. animalis* subsp. *lactis* BH13-25 | 129 |

It can be seen from Table 2 that the SNP differences between *B. animalis* subsp. *lactis* HOM2120 and the reference genome of other *B. animalis* subsp. *lactis* were all not less than 34. The results showed that *B. animalis* subsp. *lactis* HOM2120 was well distinguished from other strains of *B. animalis* subsp. *lactis* and was unique.

**Example 1-2. Isolation and Identification of *L. plantarum* HOM2217**

(1) Preparation of Artificial Gastrointestinal Fluid

Artificial gastric fluid: Into 16.4 mL of dilute hydrochloric acid (1 mol/L), 800 mL of water was added, the pH was adjusted to 3.0, and 10g of pepsin was added. The mixture was shaken well, added with water to 1000 mL, centrifuged at 5000 rpm for 5 min, the supernatant was taken, filtered with a $0.22\mu$m filter membrane for sterilization, and stored at -20°C for later use.

Artificial intestinal fluid: 6.8g of potassium dihydrogen phosphate was weighted, 500 mL of water was added, and the pH was adjusted to 6.8 with a 0.4% (0.1 mol/L) sodium hydroxide solution. Separately, 10 g of pancreatin and 3 g of pig bile salt were weighted, an appropriate amount of water was added to dissolve them. The two solutions were mixed, added with water to 1000 mL, centrifuged at 5000 rpm for 5 min. The supernatant was taken, filtered and sterilized with a 0.22 $\mu$m filter membrane, and stored at -20°C for later use.

(2) Preparation of Isolation Medium

MRS liquid medium: MRS broth medium (Cat. No. CM1163, OXOID, UK) was prepared according to the instructions, sterilized at 121°C for 15 min, and stored at 2 ~ 8°C for one week in the dark.

MRS-Cys solid medium containing bromocresol purple: 0.04g of bromocresol purple was added to each liter of MRS solid medium (Cat. No.: CM1175, OXOID, UK), and stirred well. Sterilization was performed at 121°C for 15 min, about 15 mL of the medium was poured into each petri dish in a clean bench and was allowed to solidify for later use.

(3) Isolation and Screening of *L. plantarum* HOM2217

The isolated breast milk was collected with a sterile aerobic tube and stored at low temperature in an aerobic environment. The experiment began on the day of sampling. About 1g of breast milk sample was weight and added into an aerobic tube containing 9 mL of MRS liquid medium, aerobically incubated at 37°C for 24 h. After centrifugation at 8000 rpm for 10 min, the supernatant was discarded, and 10 mL of artificial gastric fluid was added. After mixing, the mixture was incubated aerobically at 37°C for 3 h. The sample was diluted by a 10-fold dilution method, diluted to a concentration of $10^{-6}$. 100$\mu$L of each dilution was taken from the original solution to the concentration of $10^{-6}$, and spread onto MRS solid medium plates containing bromocresol purple, aerobically static incubated at 37°C for 48 h.

Single colonies with yellowing edges were picked, streaked, and purified 3-4 times until single colonies were observed. Gram staining and microscopy of colony morphology were performed simultaneously. Single colonies were then transferred to liquid medium for pure culture, preserved with glycerol, and stored at -80° C.

(4) Morphological Observation of *L. plantarum* HOM2217 Strain

*L. plantarum* HOM2217 was aerobic cultured on MRS agar medium at 37°C for 24 h. The colonies were approximately 3 mm in diameter, raised, round, the surface was moist and smooth, and were white, occasionally light yellow or dark yellow. It was observed under an optical microscope that the bacteria were rod-shaped with round-ended. The size of the bacteria was about 0.9 ~ 1.2 $\mu$m $\times$ 3 ~ 8 $\mu$m, arranged individually or in pairs, or in short chain, Gram-positive, and did not form spores.

(5) Identification of *L. plantarum* HOM2217

16S rRNA gene identification of the strain: DNA was extracted from the stored strain and amplified. PCR amplification and agarose gel electrophoresis were performed using universal primers 27F (SEQ ID NO: 7) and 1492R (SEQ ID NO: 8). Then, the gel was cut, recovered, and sequenced. The isolated strains were sequenced using 16S rDNA. According to its 16S rDNA sequence, it was aligned in the NCBI database using the BLAST tool, and the phylogenetic tree was constructed using Mega 7.0 software, as shown in FIG. 1B. As shown in FIG. 1B, the identification result was *L. plantarum,* named *L. plantarum* HOM2217. The sequence of the 16S rRNA gene was shown in SEQ ID NO: 12:

TATACATGCAGTCGAACGAACTCTGGTATTGATTGGTGCTTGC

ATCATGATTTACATTTGAGTGAGTGGCGAACTGGTGAGTAACACGTGGGAA

ACCTGCCCAGAAGCGGGGGATAACACCTGGAAACAGATGCTAATACCGCAT

AACAACTTGGACCGCATGGTCCGAGTTTGAAAGATGGCTTCGGCTATCACT

TTTGGATGGTCCCGCGGCGTATTAGCTAGATGGTGGGGTAATGGCTCACCAT

GGCAATGATACGTAGCCGACCTGAGAGGGTAATCGGCCACATTGGGACTGA

GACACGGCCCAAACTCCTACGGGAGGCAGCAGTAGGGAATCTTCCACAAT

GGACGAAAGTCTGATGGAGCAACGCCGCGTGAGTGAAGAAGGGTTTCGGC

TCGTAAAACTCTGTTGTTAAAGAAGAACATATCTGAGAGTAACTGTTCAGG

TATTGACGGTATTTAACCAGAAAGCCACGGCTAACTACGTGCCAGCAGCCG

CGGTAATACGTAGGTGGCAAGCGTTGTCCGGATTTATTGGGCGTAAAGCGA

GCGCAGGCGGTTTTTTAAGTCTGATGTGAAAGCCTTCGGCTCAACCGAAGA

AGTGCATCGGAAACTGGGAAACTTGAGTGCAGAAGAGGACAGTGGAACT

CCATGTGTAGCGGTGAAATGCGTAGATATATGGAAGAACACCAGTGGCGAA

GGCGGCTGTCTGGTCTGTAACTGACGCTGAGGCTCGAAAGTATGGGTAGCA

AACAGGATTAGATACCCTGGTAGTCCATACCGTAAACGATGAATGCTAAGTG

TTGGAGGGTTTCCGCCCTTCAGTGCTGCAGCTAACGCATTAAGCATTCCGC

CTGGGGAGTACGGCCGCAAGGCTGAAACTCAAAGGAATTGACGGGGGCCC

GCACAAGCGGTGGAGCATGTGGTTTAATTCGAAGCTACGCGAAGAACCTTA

CCAGGTCTTGACATACTATGCAAATCTAAGAGATTAGACGTTCCCTTCGGGG

ACATGGATACAGGTGGTGCATGGTTGTCGTCAGCTCGTGTCGTGAGATGTT

GGGTTAAGTCCCGCAACGAGCGCAACCCTTATTATCAGTTGCCAGCATTAA

GTTGGGCACTCTGGTGAGACTGCCGGTGACAAACCGGAGGAAGGTGGGG

ATGACGTCAAATCATCATGCCCCTTATGACCTGGGCTACACACGTGCTACAA

TGGATGGTACAACGAGTTGCGAACTCGCGAGAGTAAGCTAATCTCTTAAAG

CCATTCTCAGTTCGGATTGTAGGCTGCAACTCGCCTACATGAAGTCGGAATC

GCTAGTAATCGCGGATCAGCATGCCGCGGTGAATACGTTCCCGGGCCTTGT

ACACACCGCCCGTCACACCATGAGAGTTTGTAACACCCAAAGTCGGTGGG

GTAACCTTTTAGGAACCAGCCGC

**Example 2. Test on the Ability to Inhibit Common Pathogenic Bacteria**

(1) Indicator Bacteria Activation

The indicator strains *Escherichia coli* ATCC8739; *Staphylococcus aureus* ATCC6538; *Salmonella typhimurium* ATCC14028; *Pseudomonas aeruginosa* ATCC9027; *Listeria monocytogenes* ATCC19111 and *Clostridium difficile* ATCC9689 were all purchased from the China Center of Industrial Culture Collection. Indicator strains (*Escherichia coli* ATCC8739; *Staphylococcus aureus* ATCC6538; *Salmonella typhimurium* ATCC14028; *Pseudomonas aeruginosa* ATCC9027; *Listeria monocytogenes* ATCC19111) were inoculated into TSB medium at an inoculation amount of 1% of the total medium, and cultured aerobic at 37°C and 180 rpm for 24h for later use. *Clostridium difficile* ATCC9689 was inoculated into BHI medium at an inoculation amount of 1% of the total amount of the medium, and cultured anaerobically at 37°C for 24 h for later use.

(2) Activation of *B. animalis* subsp. *lactis*

The *B. animalis* subsp. *lactis* HOM2120 strain, cryopreserved at -80°C as prepared in Example 1, and the control strain were respectively inoculated into the sterilized MRS-Cys liquid medium described in Example 1 at an inoculation amount of 1% of the total amount of the medium. After static anaerobic culture at 37°C for 24 hours and activated twice, the strain fermentation broth was obtained. Then, the strain fermentation broth was centrifuged at 8000 rpm for 10 min, and the supernatant was taken for bacteriostatic test.

(3) Plate Preparation

The sterilized TSA medium was heated to completely melt, poured into a petri dish, placed on a horizontal platform to form an agar layer with a uniform thickness, and solidified. The indicator strains were added to the TSA medium, shaken evenly, poured into a pre-prepared TSA blank agar plate, and allowed to stand for coagulation.

(4) Bacteriostatic Test

The Oxford cup was gently placed on the plate with sterile forceps, with a certain distance between the holes, and 0.2 mL of the supernatant of the lactic acid bacteria fermentation broth to be tested was respectively added thereto. After the mixture was placed in a refrigerator at 4°C for 24 hours, cultured in an incubator at 37°C for at least 18 hours to observe the occurrence of the inhibition zone. The inhibition zone was formed and then measured with a ruler. The liquid medium (MRS-Cys) in Example 1 was used as a negative control. Each sample was tested in triplicate. The antibacterial results were shown in Table 3.

Table 3. Inhibitory Effect of *B. animalis* subsp. *lactis* HOM2120 Strain on Pathogenic Bacteria

| Sample | E. coli | Salmonella | Staphylococcus aureus | Pseudomonas aeruginosa | Listeria monocytogenes | Clostridium difficile |
|---|---|---|---|---|---|---|
| MRS-Cys (Negative Control) | - | - | - | - | - | - |
| BH13-25 | + | + | - | - | + | - |
| BH13-33 | + | + | - | - | + | - |
| HOM2120 | + | ++ | +++ | ++ | + | + |

Note: "-" no bacteriostatic activity; "+" 11-16 mm; "++" 17-22 mm; "+++" $\geq$ 23 mm

It can be seen from Table 3 that *B. animalis* subsp. *lactis* HOM2120 strain has an inhibitory effect on all 6 pathogenic bacteria. Compared with the other 2 strains of *B. animalis* subsp. *lactis,* the HOM2120 has a better inhibitory effect on *Salmonella, Staphylococcus aureus, Pseudomonas aeruginosa* and *Clostridium difficile,* indicating that it has a better ability to inhibit pathogenic bacteria.

**Example 3. Gastrointestinal transit capacity Test**

(1) Activation of Strains

The *B. animalis* subsp. *lactis* HOM2120 strain, cryopreserved at -80°C as prepared in Example 1, and the control strain were respectively inoculated into the sterilized MRS-Cys liquid medium described in Example 1 at an inoculation amount of 1% of the total amount of the medium, static anaerobic culture at 37°C for 24 hours. After bing activated twice, the strain fermentation broth was obtained.

(2) Preparation of Artificial Gastric Fluid

Into 16.4 ml of dilute hydrochloric acid, about 800 ml of water and 10g of pepsin were added, shook well, the pH was adjusted to 3.0, the water was added to 1000 ml, and filtered with a 0.2μm microporous membrane for later use.

(3) Preparation of Artificial Intestinal Fluid

6.8g of potassium dihydrogen phosphate was weighted and dissolved in 500 mL of water, and the pH was adjusted to 6.8. Separately, 10 g of trypsin and 3 g of pig bile salt were weighted, dissolved in an appropriate amount of water. The two solutions were mixed, water was added to 1000 mL, and filtered with a 0.22 um sterile membrane under a sterile environment for later use.

(4) Evaluation of Survival Capacity of Strains in Simulated Gastrointestinal tract

1 mL of activated bacterial solution of the strain to be tested was added to 9 mL of artificial gastric fluid (pH3.0), mixed well, and the number of viable bacteria was counted, and incubated at 37°C for 3 h in an incubator before counting the viable cells again. After culturing in artificial gastric fluid for 3 h, all the bacteria cells were transferred into an equal volume of artificial intestinal fluid (pH6.8), mixed well, and incubated at 37° C. The viable cell counts were performed on MRS medium at 3 h and 24 h respectively, and the survival rate was calculated by the following formula:

Survival rate in the gastric fluid for 3 hours (%) = [logCFUN1/logCFUN0] $\times$ 100%

Survival rate in the intestinal fluid for 3 hours (%)= [logCFUN2/logCFUN0] $\times$ 100%

Survival rate in the intestinal fluid for (%) = [logCFUN3/logCFUN0] $\times$ 100%

N0= the viable count of *B. animalis* subsp. *lactis* before treatment, N1= the viable count of *B. animalis* subsp. *lactis* after 3 hours of treatment with gastric fluid treatment, N2= the viable count of *B. animalis* subsp. *lactis* after 3 hours of treatment with intestinal fluid treatment, N3= the viable count of *B. animalis* subsp. *lactis* after 24 hours of treatment with intestinal fluid treatment.

Table 4. The survival rate of *B. animalis* subsp. *lactis* HOM2120 Strain in the simulated gastrointestinal fluids

| Strain Name | Survival rate in gastric fluidfor 3 hours | Survival rate in intestinal fluid for 3 hours | Survival rate in intestinal fluid Survival rate in 24 hours |
|---|---|---|---|
| BH13-33 | 100.27±0.92% | 46.33±4.73%[C] | 33.29±2.59%[C] |
| BH13-25 | 101.04±1.01% | 65.86±1.15%[B] | 42.06±1.99%[B] |
| HOM2120 | 99.13±1.17% | 98.57±1.42%[A] | 91.68±0.47%[A] |

Note: In the same column, different capital letters for the superscript indicate highly significant differences (p < 0.01).

It can be seen from Table 4 that after 3 hours of treatment with simulated gastric fluid, the survival rate of *B. animalis* subsp. *lactis* HOM2120 strain is higher than 98%. After further treatment with simulated intestinal fluid for 24 hours, the survival rate can still reach up to 91% or more, and was very significantly (p < 0.01) superior to the tolerance to intestinal fluid of the other two strains of *B. animalis* subsp. *lactis*. It indicated that *B. animalis* subsp. *lactis* HOM2120 strain had a high survival rate in the intestinal tract, laying a foundation for its colonization and effective function in the intestinal tract.

**Example 4. Intestinal Epithelial Cells Adhesion Capacity Test**

(1) Preparation of Cell Medium

Complete medium: Into high-sugar DMEM medium, 10% of inactivated fetal bovine serum (FBS) and 1% (v/v) of antibiotic (100 U/mL penicillin, 100 µg/mL streptomycin) were added, mixed and then stored at 4° C.

Incomplete medium: High-sugar DMEM medium supplemented with 10% inactivated fetal bovine serum (FBS), mixed and stored at 4° C.

(2) Cell Recovery and Culture

Human colorectal adenocarcinoma Caco-2 cells were purchased from the Cell Resource Center, Institute of Basic Medical Sciences, Chinese Academy of Medical Sciences. The cells were resuspended in a fresh culture solution, uniformly dispersed in a culture flask, cultured at 37°C under a gas condition of 5% $CO_2$ and 95% air. The culture solution was replaced every 48 h during recovery. When the cells grew well (80% confluence), Caco-2 cells were digested with trypsin-EDTA solution at 37°C. The cell concentration was adjusted to $2 \times 10^5$ cells/mL after digestion, and the cells were inoculated in a 24-well plate and cultured until the cells reached 80% confluence.

(3) Activation of *B. animalis* subsp. *Lactis*

The *B. animalis* subsp. *Lactis* HOM2120 strain, cryopreserved at -80°C as prepared in Example 1, was inoculated into the sterilized MRS-Cys liquid medium described in Example 1 at an inoculation amount of 1% of the total amount of the medium, static anaerobic cultured at 37°C for 24 hours and activated twice, and then the strain fermentation broth was obtained.

(4) Adhesion Experiment

The bacterial cells grown in the corresponding medium were collected by centrifugation at 8000 rpm for 10 min. After washing the bacterial cells with DPBS for 3 times, the bacterial cells were resuspended in the incomplete medium, and the concentration of the bacterial cells was adjusted to $10^8$ cfu/mL. 1 mL of the above bacterial suspension was added to a 24-well plate containing Caco-2 cells that have grown to a single layer, and incubated at 37°C for 2 h in a 5% $CO_2$ incubator. After incubation, the cells were washed with sterile DPBS for 3 times. The Caco-2 cells were digested with trypsin-EDTA solution at a temperature of 37°C, the number of cells and the number of viable bacteria before and after adhesion were counted. Each sample was tested in triplicate, and the results were shown in Table 5.

Table 5. Adhesion Capacity of *B. animalis* subsp. *lactis* HOM2120 Strain to Caco-2 cells

| Strain Name | Adhesion Rate (%) | Adhesion Index (CFU/Cell) |
|---|---|---|
| BH13-33 | 1.97±0.73%[B] | 1.82±0.77[b] |
| BH13-25 | 2.44±0.56%[B] | 2.48±0.31[ab] |
| HOM2120 | 3.78±0.12%[A] | 3.29±0.15[a] |

Note: In the same column, different capital letters for the superscript indicate highly significant differences ($p < 0.01$), and different lowercase letters indicate significant differences ($p < 0.05$).

The results showed that the adhesion rate of *B. animalis* subsp. *lactis* HOM2120 strain to human colon cancer cells Caco-2 was 3.78, and the adhesion index was 3.29, which was better than the other two *B. animalis* subsp. *Lactis,* and had better capacity to adhere to intestinal epithelial cells.

Adhesion index = number of bacteria after adhesion/number of cells per plate Adhesion rate = number of bacteria after adhesion/number of bacteria before adhesion

**Example 5. Test for Production Capacity of Extracellular Polysaccharide (EPS)**

The *B. animalis* subsp. *lactis* HOM2120 strain, cryopreserved at -80°C as prepared in Example 1, was inoculated into the sterilized MRS-Cys liquid medium described in Example 1 at an inoculation amount of 1% of the total amount of the medium, static anaerobic cultured at 37°C for 24 hours. After being activated twice, the strain fermentation broth was obtained. Then, the strain fermentation broth was centrifuged at 8000 rpm for 10 min, and the supernatant was taken and the extracellular polysaccharide content was detected using phenol-sulfuric acid method. The MRS-Cys liquid medium described in Example 1 was used as a negative control. Each sample was performed in triplicate, and the data was analyzed using one-way ANOVA with SPSS software. The results were shown in Table 6.

Table 6. Extracellular polysaccharide (EPS) production capacity of *B. animalis* subsp. *lactis* HOM2120 Strain

| Sample | EPS Production (mg/L) |
|---|---|
| MRS- Cys (negative control) | 190±2D |
| BH13-25 | 289±3C |
| BH13-33 | 269±5B |
| HOM2120 | 497±3A |

Note: In the same column, different capital letters for the superscript indicate highly significant differences (p < 0.01).

It can be seen that compared with other two strains of *B. animalis* subsp. *lactis, B. animalis* subsp. *lactis* HOM2120 strain had a stronger capacity to produce extracellular polysaccharide (EPS).

**Example 6. Lactic Acid and Short Chain Fatty Acid Production Ability Test**

The *B. animalis* subsp. *lactis* HOM2120 strain, cryopreserved at -80°C as prepared in Example 1, was inoculated into the sterilized MRS-Cys liquid medium described in Example 1 at an inoculation amount of 1% of the total amount of the medium, static anaerobic cultured at 37°C for 24 hours. After being activated twice, the strain fermentation broth was obtained. Then the broth was centrifuged at 8000 rpm for 10 min, and the supernatant was taken the contents of lactic acid was determined by gas chromatography and short-chain fatty acids. The MRS-Cys liquid medium described in Example 1 was used as a negative control, the other two strains of *B. animalis* subsp. *lactis* were used as positive controls. Each sample was performed in triplicate. The data were analyzed by SPSS25.0 software for one-way ANOVA analysis and Duncan's multiple comparison.

Table 7. Lactic acid and short-chain fatty acid production capacity of *B. animalis* subsp. *lactis* HOM2120 Strain

| Sample | Lactic Acid (g/L) | Acetic Acid (g/L) | Formic Acid (g/L) | Propionic Acid (mg/L) | Butyric Acid (mg/L) | Isobutyric Acid (mg/L) |
|---|---|---|---|---|---|---|
| MRS-Cys (Negative Control) | 0.54±0.01C | 2.13±0.01C | 0.16±0.00B | 11.4±0.2B | 12.3±0.3D | 10.0±0.2C |
| BH13-25 | 0.93±0.04B | 4.07±0.10B | 0.19±0.01B | 19.7±0.1B | 14.4±0.1C | 10.4±0.1BC |
| BH13-33 | 0.92±0.01B | 4.04±0.03B | 0.17±0.01B | 20.3±0.4B | 15.2±0.2B | 10.8±0.1B |
| HOM2120 | 1.76±0.01A | 4.36±0.09A | 1.05±0.05A | 21.6±0.1A | 16.3±0.3A | 12.0±0.3A |

Note: In the same column, different capital letters for the superscript indicate highly significant differences (p < 0.01).

It can be seen from Table 7 that compared with other two strains of *B. animalis* subsp. *lactis* strain, *B. animalis* subsp. *lactis* HOM2120 strain had a stronger capacity to produce lactic acid and short-chain fatty acids (acetic acid, formic acid, propionic acid, butyric acid and isobutyric acid).

**Example 7. In Vitro Cytokine Secretion Promoting Test**

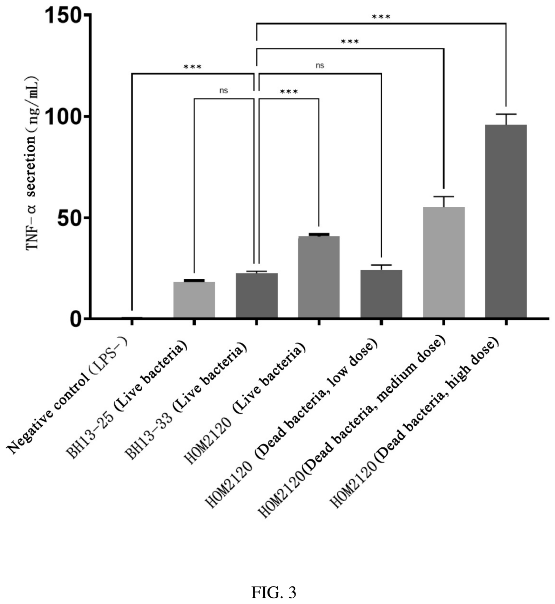

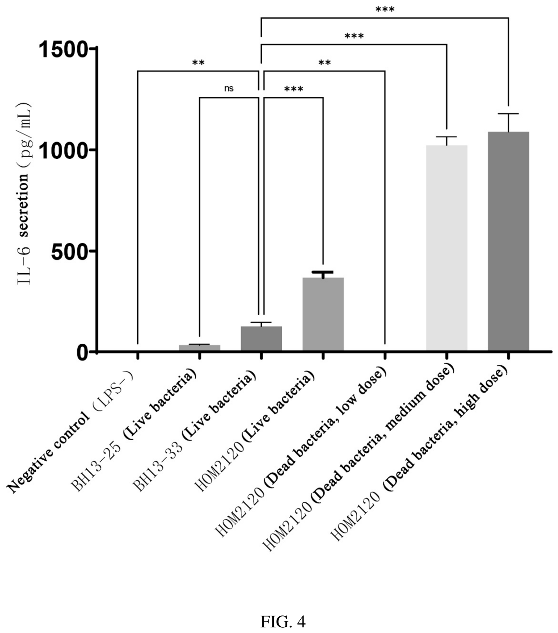

*The B. animalis* subsp. *lactis* HOM2120 strain cryopreserved at -80°C as prepared in Example 1, was inoculated into the sterilized MRS-Cys liquid medium described in Example 1 at an inoculation amount of 1% of the total amount of the medium, static anaerobic cultured at 37°C for 24 hours. After being activated twice, it was used for viable counting and cell experiments. It was centrifuged at 8000 rpm for 10 min to collect bacterial cells. The DMEM complete medium without antibiotics was used to adjust the concentration of bacterial cells to working level (about $5.0 \times 10^5$ CFU/mL) as a live bacteria group. Moreover, after the bacterial cells were collected, heated at 100°C for 30 min, and then the bacterial count was adjusted to about $5.0 \times 10^5$ CFU/mL as the low-dose group of dead bacteria, to about $5.0 \times 10^6$ CFU/mL as the medium dose group of dead bacteria, and to about $5.0 \times 10^7$ CFU/mL as the high-dose group of dead bacteria. The mouse macrophages RAW264.7 (about $5 \times 10^5$ cell/mL) were added to a 24-well culture plate, with 1 mL per well. After 2 h of adhesion, the medium was discarded, and 1 mL of bacteria-containing medium was added to each well. A blank control group was set, with 1 mL of DMEM medium was added. After 24 h of co-culture, the cell supernatant was collected, and the contents of TNF-α and IL-6 in the cell supernatant were determined by enzyme-linked immunosorbent assay (ELISA) according to the instructions of the kit. The MRS-Cys liquid medium described in Example 1 was used as a negative control, the same dose of viable strains of *B. animalis* subsp. *Lactis* BH13-25 and BH13-33 were used as a positive control. Each sample was prepared in triplicate. Each sample was prepared in triplicate. The data were analyzed by using GraphPad prism9 software for one-way ANOVA and Dennett's t-test (pairwise comparison of means between multiple experimental groups and a control group). The results were shown in FIG. 3 and FIG. 4.

As can be seen from FIG. 3 and FIG. 4, compared with *B. animalis* subsp. *lactis* BH13-33, both live bacteria and dead bacteria of *B. animalis* subsp. *lactis* HOM2120 can significantly (p < 0.001) improve the secretion of TNF-α and IL-6 by RAW264.7 cells. Under the same dosage condition, live bacteria were superior to dead bacteria; with the increase of the dosage, the immunity was enhanced. It indicated that *B. animalis* subsp. *lactis* HOM2120 had potential immunomodulatory capacity.

## Example 8. Antibiotic Susceptibility Test

The drug susceptibility test was carried out according to ISO10932-2010, "Milk and milk products - Determination of the minimal inhibitory concentration (MIC) of antibiotics applicable to bifidobacteria and non-enterococcal lactic acid bacteria (LAB)", established by the International Organization for Standardization.

(1) Preparation of Medium

MRS-Cys liquid medium: preparation method is the same as in Example 1

LSM-Cys liquid medium: 21.06g of Iso-Sensitest medium (Cat. No. CM0473B, OXOID, UK) was weighed, 5.2 g of MRS broth and 0.3 g of L-cysteine hydrochloride were weighed, added with water to 0.5 L, the pH was adjusted to 6.85 ± 0.1, and sterilized at 121°C for 15 min, and the pH should be 6.7 ± 0.1 for later use, and stored at 2 - 8°C for one week in the dark.

(2) Activation and Proliferation of *B. animalis* subsp. *lactis* Strain

The *B. animalis* subsp. *lactis* HOM2120 strain, cryopreserved at -80°C as prepared in Example 1, was inoculated into the sterilized liquid medium (MRS-Cys) described in Example 1 at an inoculation amount of 1% of the total amount of the medium, static anaerobic cultured at 37°C for 24 hours. After being activated twice, the strain fermentation broth was obtained. The activated *B. animalis* subsp. *lactis* HOM2120 strain was propagated on MRS-Cys agar medium, and static anaerobic cultured at 37°C for 48 hours.

(3) Preparation of Antibiotic Microdilution Plate

0.0512g of antibiotic was weighed, into which 10 mL of solvent was added, wherein chloramphenicol and erythromycin were dissolved in ethanol (without filtration), ampicillin was dissolved in phosphate buffer (pH 8.0, 0.1 mol/L), and other antibiotics were dissolved in water. After shaking to dissolve, filtered with a 0.22μm filter membrane, sub-packaged into EP tubes, with a concentration of 5120 μg/mL (5.12 mg/mL), and stored at -20°C for later use. The antibiotic stock solution was diluted with water (ampicillin with phosphate buffer) to a suitable concentration range. 50μL of diluent was added to the wells of the microdilution plate.

(4) Preparation of Bacterial Suspensions

Individual colonies were picked from agar plates. The obtained colonies were suspended in sterile culture tubes containing 2 mL to 5 mL of sterile saline. The obtained colonies were suspended in pre-reduced LSM-Cys liquid medium. Colonies were suspended until the solution turbidity reached McFarland Standard 1 or the optical density at 625 nm was 0.16 - 0.2 using the spectrophotometer. The suspension was approximately equivalent to $3 \times 10^8$ CFU/mL. The bacterial suspension was diluted with the recommended medium and the bacterial suspension was diluted 500-fold with MRS-Cys liquid medium, as the antibiotic solution will dilute the medium by two times. The diluted bacterial suspension was dispensed within 30 minutes after the preparation. When the freeze plate is used, the frozen antibiotic solution was thawed under anaerobic conditions immediately prior to use. 50μL of the diluted suspension was dispensed into each well of the microdilution plate (about $3 \times 10^4$ CFU/well). The plates were incubated at 37°C under static anaerobic conditions for 48 h. When an anaerobic jar was used, the plates were capped between each plate to create a homogeneous environment in the anaerobic jar. Each experiment was repeated three times, with both positive control group and a negative control group were set. The positive control wells did not contain antibiotics, but included the test strains and medium containing solvents to dissolve the highest concentrations of antibiotics. Negative control wells did not contain the test strain and antibiotics, but included medium.

(5) Reading MIC Results

After 48 hours of incubation, the MIC was visually read. After incubation, the negative control wells were checked for visible bacterial growth. If contamination was found, all data generated by the relevant strain was rejected. Note: If there was no growth in the positive control well, it indicated that the tested strain was sensitive to the solvent used to dissolve the antibiotic. In this case, reading the MIC for that particular antibiotic was meaningless. If the negative and positive controls were normal, the growth of bacteria in each antibiotic was determined visually by comparing them with the positive control. Preferably, the microdilution plate was placed on the top of the frame of the magnifying glass, and the table lamp providing indirect light for easy reading. The growth of bacteria was easily detected under the magnifying glass as sediment at the bottom of the well. Any series of wells in which growth discontinuities were observed were discarded (e.g., grown at 16 $\mu$g/mL and 64 $\mu$g/mL, but not at 32$\mu$g/mL). The endpoint was defined as the lowest antibiotic concentration at which no growth was visually observed. The concentration is the MIC of that antibiotic for that specific strain. Each sample was tested in triplicate, and *Lactobacillus plantarum* ATCC14917 was used as a positive control strain. The antibiotic susceptibility results of *B. animalis* subsp. *lactis* HOM2120 strain were shown in Table 8. The MIC results of *Lactobacillus plantarum* ATCC14917 were consistent with those shown in the ISO10932-2010 appendix, indicating that the experimental method was accurate. According to the antibiotic resistance standards for bacterial strains used in food established by the European Food Safety Authority (EFSA) in 2012, the antibiotic resistance standard for using strains in food was formulated, and it can be seen that B. *animalis* subsp. *lactis* HOM2120 was sensitive to erythromycin, chloramphenicol, vancomycin, ampicillin, clindamycin, streptomycin, and kanamycin. Therefore, the product of *B. animalis* subsp. *lactis* HOM2120 was relatively safe.

Table 8. Antibiotic susceptibility results of *B. animalis* subsp. *lactis* HOM2120 Strain

| Antibiotics | Judgment Criteria | | *B. animalis* subsp. *lactis* HOM2120 | | *L. plantarum* ATCC14917 (Control Strain) | |
|---|---|---|---|---|---|---|
| | Sensitivity (S) | Resistance (R) | MIC Value | Results | MIC Value | Results |
| Erythromycin | ≤1 | >1 | 0.5±0 | S | 1±0 | S |
| Chloramphenicol | ≤4 | >4 | 1.6±0.5 | S | 0.83±0.29 | S |
| Tetracycline | ≤8 | >8 | 16±0 | R | 8±0 | S |
| Vancomycin | ≤2 | >2 | 0.5±0 | S | 128±0 | / |
| Ampicillin | ≤2 | >2 | 0.5±0 | S | 0.5±0 | S |
| Clindamycin | ≤1 | >1 | 0.063±0 | S | 0.5±0 | S |
| Gentamicin | ≤64 | >64 | 107±37 | R | 4±0 | S |
| Streptomycin | ≤128 | >128 | 64±0 | S | 128±0 | / |

**Example 9. Preparation Process 1 of Active Bacterial Powder of *B. animalis* subsp. *lactis* HOM2120 Strain**

(1) Culture of Strain

The *B. animalis* subsp. *lactis* HOM2120 strain, cryopreserved at -80°C as prepared in Example 1, was inoculated into the sterilized MRS-Cys liquid medium at an inoculation amount of 1% of the total amount of the medium, and cultured at 37°C for 16-24 hours. After two such subcultures, activated seed culture solution was obtained. The seed culture solution was inoculated into a fermentation medium M416 at an inoculation amount of 1% of the total amount of the medium, and cultured to constant temperature anaerobically at 37°C with a rotation speed of 50 rpm. During the fermentation, a sodium hydroxide solution was automatically added to maintain the pH constant at 5.0. After fermentation for 16-24 hours, it was transferred to another tank to obtain a high-density culture solution of *B. animalis* subsp. *lactis* HOM2120 strain, with a viable count of up to 22.4 billion CFU/mL. The formulation of the fermentation medium M416 is shown was Table 9.

Table 9. Formulation of Fermentation Medium M416

| Medium Components | Content (g/L) |
| --- | --- |
| Glucose | 50 |
| Yeast Extract | 80 |
| Magnesium Sulfate | 0.4 |
| Manganese Sulfate | 0.2 |
| Dipotassium hydrogen phosphate | 4 |
| Potassium Dihydrogen Phosphate | 4 |
| Tween-80 | 2 |
| Calcium Chloride | 0.1 |
| L-Cysteine Salt | 1 |

(2) Preparation of Lyoprotectant

A protectant containing 100 g/L skim milk powder, 50 g/L sucrose, 2 g/L vitamin C, and 1 g/L L-cysteine salt was prepared by mixing sterile water with raw materials of the protectant.

(3) Freeze-Drying

The fermentation broth of *B. animalis* subsp. *lactis* HOM2120, after culture, was centrifuged at 6500 rpm for 15 min at 2-8°C. The supernatant was discarded, the bacterial sludge was collected. The bacterial sludge was washed with 0.9% sterile physiological saline for 1-2 times. The washed bacterial sludge was mixed with the above-mentioned protectant, to achieve a bacterial concentration reached $10^{10}$ CFU/mL or more in the mixed bacterial solution. It was then freeze-dried in a freeze dryer, pre-frozen at -40°C for 4 hours, vacuumized, the temperature was raised to - 12 °C, and dried for 22 hours in a primary drying; then the temperature was raised to 35°C, and dried for 7 hours in a secondary drying. After freeze-drying, the bacterial cake was pulverized by a fine grinder to obtain the freeze-dried bacterial powder. The viable count of the freeze-dried bacterial powder reached $4.3 \times 10^{11}$ CFU/g.

**Example 10. Preparation Process 2 of Active Bacterial Powder of *B. animalis* subsp. *lactis* HOM2120**

(1) Culture of Strain

The *B. animalis* subsp. *lactis* HOM2120 strain, cryopreserved at -80°C as prepared in Example 1, was inoculated into the sterilized MRS-Cys liquid medium at an inoculation amount of 3% of the total amount of the medium, and cultured at 37°C for 16-24 hours. After two such subcultures, activated seed culture solution was obtained. The seed culture solution was inoculated into a fermentation medium M453 at an inoculation amount of 3% of the total amount of the medium, and cultured to constant temperature anaerobically at 37°C with a rotation speed of 50 rpm. During the fermentation, a sodium hydroxide solution was automatically added to maintain the pH constant at 5.5. After fermentation for 16-24 hours, it was transferred to another tank to obtain a high-density culture solution of *B. animalis* subsp. *lactis* HOM2120, with a viable count of up to 15.3 billion CFU/mL.

Table 10. The formulation of the fermentation medium M453

| Medium Components | Content (g/L) |
| --- | --- |
| Glucose | 30 |
| Yeast Extract | 70 |
| Magnesium Sulfate | 0.8 |
| Manganese Sulfate | 0.4 |
| Dipotassium hydrogen phosphate | 1 |
| Potassium Dihydrogen Phosphate | 1 |
| Tween-80 | 1 |
| Calcium Chloride | 0.4 |
| L-Cysteine Salt | 0.5 |

(2) Preparation of Lyoprotectant

A protectant containing 150 g/L skim milk powder, 20 g/L sucrose, 1 g/L vitamin C, and 0.5 g/L L-sodium glutamate was prepared by mixing sterile water with raw materials of the protectant.

(3) Freeze-Drying

The fermentation broth of *B. animalis* subsp. *lactis* HOM2120, after culture, was centrifuged at 6500 rpm for 15 min at 2-8°C. The supernatant was discarded, and the bacterial sludge was collected. The bacterial sludge was washed with 0.9% sterile physiological saline for 1-2 times. The washed bacterial sludge was mixed with the above-mentioned protectant, to achieve a bacterial concentration of $10^{10}$ CFU/mL or more in the mixed bacterial solution. It was then freeze-dried in a freeze dryer, pre-frozen at -35°C for 5 hours, vacuumized, the temperature was raised to -10°C, and dried for 15 hours in a primary drying; then the temperature was raised to 30°C, and dried for 10 hours in a secondary drying. After freeze-drying, the bacterial cake was pulverized by a fine grinder to obtain the freeze-dried bacterial powder. The viable count of the freeze-dried bacterial powder reached $3.7 \times 10^{11}$ CFU/g.

**Example 11. Preparation Process of Dead Bacterial Powder of *B. animalis* subsp. *lactis* HOM2120 Strain**

(1) Culture of Strain

The strain culture was as described in Example 9 or Example 10.

(2) Preparation of Bacterial Powder

The fermentation broth of *B. animalis* subsp. *lactis* HOM2120 obtained by high-density fermentation was treated at 100°C for 10 minutes, 100g of maltodextrin excipient was added per liter of the fermentation broth, and dried by a spray dryer. The number of dead bacteria in the dead bacterial powder was $2.2 \times 10^{11}$ CFU/g when counted under a microscope using a hemocytometer.

**Example 12. Animal Test 1 to Enhanced Immunity**

(1) Experimental animals and grouping: 192 female SPF-grade KM mice (18-20g) bred in Kunming by Beijing Huafukang Biotechnology Co., Ltd were selected. After animal adaptation and observation, they were randomly divided into four batches, with four groups per batch and 12 mice per group. The experimental groups were divided into three dose groups: a low-dose group ($5 \times 10^9$ CFU/Kg BW), a medium-dose group ($2.5 \times 10^{10}$ CFU/Kg BW) and a high-dose group ($5 \times 10^{10}$ CFU/Kg BW). The mice were administered with the active bacterial powder of *B. animalis* subsp. *lactis* HOM2120 strain prepared in Example 9 by gavage with physiological saline as the solvent, which was administered orally once a day. Various indicators were measured after 32 days of continuous gavage. Mice were administered with a volume of 10 mL/kg BW via gavage, while the control group was administered with a high dose of physiological saline solution containing the same mass of bacterial powder excipients via gavage. Each dose group was given maintenance feed. Experimental batch one underwent carbon clearance test; experimental batch two underwent organ/body weight ratio measurement and a delayed-type hypersensitivity experiment; experimental batch three underwent mouse peritoneal macrophage phagocytosis of chicken red blood cells experiments; and experimental batch four underwent experiment of concanavalin A (ConA)-induced transformation of mouse lymphocyte.

(2) Experimental Methods

A. Determination of Organ/Body Weight Ratio

After weighing, the mice were sacrificed by cervical dislocation. The spleen and thymus were taken, fascia was removed, blood stains were blotted from the surface of the organ with filter paper, and then weighed. The spleen/body weight ratio and thymus/body weight ratio were calculated.

B. Experiment of Delayed Type Hypersensitivity (DTH) Experiment (Plantar Thickening Method)

The sheep blood was taken and washed 3 times with physiological saline, and each mouse was intraperitoneally injected with 0.2 mL of 2% (v/v, prepared with physiological saline) sheep red blood cell (SRBC) suspension (2000 r/min, 10 min). Four days after sensitization, the thickness of the left hind paw plantar region was measured. Then, 20 μL of 20% (v/v, prepared with physiological saline) SRBC suspension was injected subcutaneously at the measurement site, and the thickness of the left hind paw plantar region was measured 24h after injection. The above two measurements were taken three times at the same site, and the average value was used. The difference in plantar thickness before and after attack was used to represent the degree of DTH. The difference of the test sample group was significantly higher than that of the control group, indicating a positive result for this experiment.

C. ConA-Induced Mouse Lymphocyte Transformation Test (MTT Method)

The spleen was aseptically removed and placed in a small petri dish containing an appropriate amount of sterile Hank's solution. The spleen was gently ground with tweezers to prepare a single-cell suspension. The suspension was filtered through a 200-mesh sieve to prepare a cell suspension. It was washed twice with Hank's solution and centrifuged for 5 min each time (1000 r/min). The cells were then suspended in 1m of complete medium, counted by microscopy, and the cell concentration was adjusted to $3 \times 10^6$ cells/mL. The spleen cell suspension was then added to two wells of a 24-well culture plate at 1.0 mL per well. One well contained 75 μL of ConA solution (equivalent to 7.5 μg/mL), and the other well served as a control. The plate was cultured in a carbon dioxide incubator with 5% $CO$, at 37°C for 72 h. Four hours before the end of the culture, 0.7 mL of supernatant was gently aspirated from each well, and 0.7 mL of RPMI1640 culture solution (without fetal bovine serum) was added. Simultaneously, 50 μL of MTT (5 mg/mL) per well was added, and incubated for 4 h. After the incubation was completed, 1.0 mL of acidic isopropanol was added to each well, and the mixture was pipetted and mixed to completely dissolve the purple crystals. This solution was then moved into a cuvette and measured colorimetrically at 570 nm using a spectrophotometer. The proliferation capacity of lymphocytes was obtained by subtracting the optical density value of the well containing ConA from the optical density value of the well without ConA. The optical density difference of the test sample group was significantly higher than that of the control group, which indicated that the result of the experiment is positive.

D. Antibody - Forming Cell (PFC) Test (Jerne's Modified Slide Method)

The sheep blood was collected and washed 3 times with physiological saline. Each mouse was intraperitoneally injected with 0.2 mL of 2% (v/v, prepared with physiological saline) SRBC suspension. Five days after SRBC immunization, mice were sacrificed by cervical dislocation. The spleen was taken out and placed in a small petri dish containing an appropriate amount of sterile Hank's solution. The spleen was gently ground to prepare a cell suspension. The suspension was centrifuged (1000 r/min) for 5 min, washed twice with Hank's solution, and finally suspended cells in 8.0 mL of Hank's solution. After the agarose was heated and dissolved, it was mixed with an equal amount of 2-fold concentration of Hank's solution, and dispensed into small test tubes, 0.5 mL of each tube. Then, 50 μL of 10% (v/v, prepared with SA solution) SRBC suspension and 8 μL of spleen cell suspension were added to the tubes, mixed rapidly, poured onto glass slides coated with a thin layer of agarose, parallel slides were prepared. After the agarose solidified, the slides were horizontally placed on a slide holder, placed in a carbon dioxide incubator and incubated at 37°C for 1 h. Then, complement diluted with SA buffer (1 : 8) was added into the grooves of the slide holder, and continued incubation for 1.5 h. The number of hemolytic plaques was counted. It was expressed by the number of plaques/whole splenocytes. The number of plaques in the test sample group was significantly higher than that in the control group, which indicates that the result of the experiment was positive.

E. Determination of Half Hemolysis Value (HC50)

Sheep blood was collected and washed three times with physiological saline. Each mouse was immunized by intraperitoneal injection of 0.2 mL of 2% (v/v, prepared with physiological saline) SRBC suspension. After 5 days, the eyeballs were removed, and blood was collected in a centrifuge tube. After standing for about 1 hour, the clotted blood was peeled off from the tube wall to allow the serum to be fully separated, centrifuged at 3000 r/min for 10 min and the serum was collected. The serum was diluted 300-fold with SA buffer, 1.0 mL of which was taken and placed in a test tube, and 0.5 mL of 10% (v/v, prepared with SA buffer) SRBC suspension and 1.0 mL of complement (diluted with SA buffer at 1 : 8) were added sequentially. A control tube without serum (replaced with SA buffer) was also provided. After heat preservation in a 37°C constant-temperature water bath for 15 min, the reaction was terminated in an ice bath. The control tube was centrifuged at 2000 r/min for 10 min, 1.0 mL of supernatant was collected, and 3.0 mL of Hb diluent was added. At the same time, 0.25 mL of 10% SRBC suspension (v/v, prepared with SA buffer) was taken, added with Hb diluent to a final volume of 4.0 mL in another test tube, mixed well. After standing for 10 minutes, the optical density value of each tube was measured at 540 nm using the control tube as a blank. The amount of hemolysin was expressed as half hemolysis value (HC50), which was calculated according to the following formula: half hemolysis value of sample = sample optical density value / SRBC optical density value at half hemolysis $\times$ dilution factor. The HC50 of the test sample group is significantly higher than the HC50 of the control group, which indicates that the result of the experiment is positive.

F. Mouse Carbon Clearance Test

4-fold diluted Indian ink (0.05 mL/10 gBW) was injected into the tail vein of mice according to body weight. After the ink was injected, timing was performed immediately. After 2 min and 10 min of ink injection, 20 $\mu$L of blood was taken from the venous plexus of angular vein and added to 2.0 mL of 0.1% $Na_2CO_3$ solution, respectively. A spectrophotometer was used to measure optical density (OD) at a wavelength of 600 nm, and a 0.1% $Na_2CO_3$ solution was used as a blank control. Mice were sacrificed, and livers and spleens were weighed. The phagocytic index (a) represents the mouse's carbon clearance capacity. (a) is calculated as follows:

$$k = (\lg OD1 - \lg OD2)/(t2 - t1), a = \text{body weight} \div (\text{liver weight} + \text{spleen weight}) \times k^{1/3}$$

The phagocytic index of the test sample group was significantly higher than that of the control group, which indicated that the result of the experiment was positive.

G. Experiment of Mouse Intraperitoneal macrophages phagocytosing Chicken Red Blood Cells (Semi-In-Vivo Method)

Mice were intraperitoneally injected with 1 mL of 20% (v/v, prepared with physiological saline) chicken red blood cell (2000 r/min, 10 min) suspension. After 30 min intervals, the mice were sacrificed by cervical dislocation, and fixed in supine position on a mouse plate. 2 mL of physiological saline was injected intraperitoneally, and the abdomen was gently massaged for 20 times. 1 mL of peritoneal macrophage washing solution was taken and dropped on two glass slides respectively, placed in an enamel box padded with wet gauze, and incubated in an incubator at 37°C for 30 min. After incubation, the slides were rinsed in physiological saline to remove unattached cells. The slides were air-dried, fixed with 1:1 acetone methanol solution, stained with Giemsa-phosphate buffer, rinsed with distilled water, and dried in air. Macrophages were counted under an oil immersion microscope, with 100 macrophages counted per slide. The phagocytic rate and phagocytic index were calculated using the following formulas:

Phagocytic percentage (%) = number of macrophages phagocytosing chicken red blood cells / number of macrophages counted $\times$ 100%

Phagocytic index = Total number of phagocytosed chicken red blood cells / number of macrophages counted

The obtained phagocytic percentage was then converted using the following formula: $X = Sin^{-1}\sqrt{P}$, where P was the phagocytic percentage, expressed as a decimal. The obtained data were quantitative data. The phagocytic percentage and phagocytic index of the test sample group were significantly higher than those of the control group, which indicated that the result of the experiment was positive.

H. Determination of NK Cell Activity (Lactate Dehydrogenase LDH Assay)

The target cells YAC-1 were subcultured at 24h before the experiment. The cells were washed 3 times with Hank's solution before use, and the cell concentration was adjusted to $4 \times 10^5$ cells/mL with RPMI1640 complete medium (containing 10% fetal bovine serum). The tested mice were sacrificed by cervical dislocation. The spleen was taken out aseptically, and spleen cell suspensions were prepared, washed twice with Hank's solution, and centrifuged for 10 min each time (1000 r/min). The supernatant was discarded and the cell pellet was resuspended, 0.5 mL of sterilized water was added for 20 seconds, red blood cells were lysed, then 0.5 mL of 2-fold Hank's solution and 8.0 mL of Hank's solution were added. It was centrifuged at 1000 r/min for 10 min, resuspended with 1.0 mL of RPMI1640 complete culture solution (containing 10% fetal bovine serum), and counted by microscopic examination, and the cell concentration was adjusted to $2 \times 10^7$ cells/mL with the RPMI1640 complete culture solution. The effector-to-target ratio was at 50 : 1. 100 $\mu$L of the target cells and 100 $\mu$L of the effector cells were taken and added to a U-shaped 96-well culture plate. 100 $\mu$L of the target cells and 100 $\mu$L of the medium were added to the target cell natural release wells, and 100 $\mu$L of the target cells and 100 $\mu$L of the 1% NP40 were added to the target cell maximum release wells. Each of these wells were provided with three parallel wells. The cells were cultured in a carbon dioxide incubator at 37°C and 5% $CO_2$ for 4 h. The 96-well plate was centrifuged at 1500 r/min for 5 min, 100 $\mu$L of supernatant from each well was pipetted and placed in a flat-bottomed 96-well culture plate, 100 $\mu$L of LDH matrix solution was added to react for 10 min. Then, 30 $\mu$L of 1 mol/L HCl solution was added to each well to terminate the reaction. The optical density (OD) was measured at 490 nm using a microplate reader. NK cell activity was calculated: NK cell activity (%) = (OD of reaction well - OD of natural release well)/(OD of maximum release well - OD of natural release well) $\times$ 100%

The obtained NK cell activity was converted using the following formula: $X = \mathrm{Sin}^{-1}\sqrt{P}$, where P was the NK cell activity expressed as a decimal. The obtained data were quantitative data. The NK cell activity of the test sample group is significantly higher than that of the control group, which indicated that the result of the experiment was positive.

(3) Data Processing

Data processing was performed with SPSS software. The analysis of variance was adopted, but a homogeneity of variance test was performed first according to the ANOVA procedure. If the variance were homogeneous, the F value was calculated. If the F value is < F0.05, the conclusion was: there was no significant difference between the averages of each group. If the F value is $\geq$ F0.05, P $\leq$ 0.05, Duncan's multiple comparison method was used for statistical analysis. For non-normal or unequal variance data, appropriate variable transformations were performed until the data met the requirements of normality or homogeneity of variance. The transformed data were used for statistics analysis. If the transformations still did not achieve normality or homogeneity of variance, the rank-sum test was used for statistical analysis.

(4) Experimental Results

Table 11

| Group | n | Initial Body Weight (g) Batch 1 | Weight (g) Batch 2 | Final Body Weight (g) Batch 1 | Weight (g) Batch 2 |
|---|---|---|---|---|---|
| G1 (control group) | 12 | 21.2$\pm$1.1 | 21.1$\pm$1.0 | 36.2$\pm$2.5 | 36.3$\pm$2.5 |
| G2 (HOM2120 low-dose group) | 12 | 21.2$\pm$0.8 | 21.2$\pm$0.7 | 37.1$\pm$1.9 | 36.8$\pm$2.4 |
| G3 (HOM2120 medium-dose group) | 12 | 21.2$\pm$0.6 | 21.2$\pm$0.9 | 36.0$\pm$2.1 | 36.3$\pm$2.6 |
| G4 (HOM2120 high-dose group) | 12 | 21.2$\pm$1.0 | 21.2$\pm$0.9 | 36.0$\pm$2.4 | 36.0$\pm$2.7 |
| Group | n | Initial Body Weight (g) Batch 3 | Weight (g) Batch 4 | Final Body Weight (g) Batch 3 | Weight (g) Batch 4 |
| G1(control group) | 12 | 21.1$\pm$1.1 | 21.1$\pm$1.1 | 37.0$\pm$2.3 | 36.9$\pm$2.4 |
| G2 (HOM2120 low-dose group) | 12 | 21.2$\pm$0.9 | 21.2$\pm$0.6 | 37.9$\pm$3.3 | 35.1$\pm$2.4 |
| G3 (HOM2120 medium-dose group) | 12 | 21.2$\pm$0.8 | 21.2$\pm$0.9 | 35.7$\pm$3.1 | 36.6$\pm$2.6 |
| G4 (HOM2120 high-dose group) | 12 | 21.2$\pm$0.7 | 21.2$\pm$0.9 | 36.5$\pm$2.6 | 36.1$\pm$3.5 |
| Note: Comparison between each group and the negative control group, p > 0.05 | | | | | |

It can be seen from the results in Table 11 that, compared with the control group, there was no significant difference (p > 0.05) in body weight before and after the experiment in the high, medium, and low dose groups of *B. animalis* subsp. *lactis* HOM2120 strain. This indicated that the active bacterial powder of *B. animalis* subsp. *lactis* HOM2120 strain had no effect on the body weight of the mice.

Table 12. The effect of bacterial powder of *B. animalis* subsp. *lactis* HOM2120 strain on the organ/body weight ratio in mice ( x$\pm$S).

| Group | n | Spleen/Body (mg/g) | Thymus/Body (mg/g) |
|---|---|---|---|
| G1(control group) | 12 | 4.67$\pm$1.25 | 3.67$\pm$0.58 |
| G2 (HOM2120 low-dose group) | 12 | 4.23$\pm$0.51 | 3.58$\pm$0.75 |
| G3 (HOM2120 medium-dose group) | 12 | 4.46$\pm$0.90 | 3.68$\pm$0.81 |
| G4 (HOM2120 high-dose group) | 12 | 4.23$\pm$0.86 | 3.64$\pm$0.60 |
| Note: Comparison between the test group and the negative control group, compared, p > 0.05 | | | |

As can be seen from the results in Table 12, compared with the control group, there was no significant difference (p > 0.05) in spleen/body weight and thymus/body weight of mice in the high, medium and low dose groups of *B. animalis* subsp. *lactis* HOM2120 strain active bacterial powder. This indicated that the *B. animalis* subsp. *lactis* HOM2120 strian active bacterial powder had no effect on the spleen and thymus weights of mice.

Table 13. The effects of bacterial powder of *B. animalis* subsp. *lactis* HOM2120 strain on mice DTH induced by sheep red blood cells (SRBC) and on proliferation capacity of mouse spleen lymphocytes induced by ConA ( x±S)

| Group | n | Cell Immune Function Plantar Thickness Difference (mm) | Lymphocyte Proliferation Capacity (ΔOD Value) |
|---|---|---|---|
| G1(control group) | 12 | 0.65±0.35 | 0.047±0.017 |
| G2 (HOM2120 low-dose group) | 12 | 1.07±0.25** | 0.060±0.028 |
| G3 (HOM2120 medium-dose group) | 12 | 1.00±0.28* | 0.061±0.030 |
| G4 (HOM2120 high-dose group) | 12 | 1.01±0.41* | 0.086±0.032** |

[00234] Note: * indicates p < 0.05 compared to the control group; ** indicates p < 0.01 compared to the control group; *** indicates p < 0.001 compared to the control group.

As can be seen from the results in Table 13, compared with the control group, in the *B. animalis* subsp. *lactis* HOM2120 strain active bacterial powder, the active bacterial powder in the high dose group can significantly improve the degree of mouse paw plantar thickening induced by sheep red blood cell (p < 0.05); and the active bacterial powder in the low dose group can significantly improve the degree of mouse paw plantar thickening induced by sheep red blood cell (p < 0.01). High dose group of active bacterial powder of *B. animalis* subsp. *lactis* HOM2120 strain can significantly improve the proliferation capacity of mouse spleen lymphocytes induced by ConA (p < 0.001). This indicates that active bacterial powder of *B. animalis* subsp. *lactis* HOM2120 strain significantly enhances the cellular immune function of mice.

Table 14. The effect of bacterial powder of *B. animalis* subsp. *lactis* HOM2120 Strain on the number of antibody-producing cells and the half hemolysis value of serum ( x̄±S)

| Group | Humoral Immune Function | |
|---|---|---|
| | Antibody-Producing Cell Experiment | Serum Hemolysin Experiment |
| | The number of hemolytic plaques (×10³/whole spleen) | Half hemolysis value |
| G1(control group) | 141±25 | 161±43 |
| G2 (HOM2120 low-dose group) | 140±32 | 164±61 |
| G3 (HOM2120 medium-dose group) | 155±31 | 169±64 |
| G4 (HOM2120 high-dose group) | 161±28 | 178±46 |

Note: * indicates p < 0.05 compared to G1 group; ** indicates p < 0.01 compared to G1 group; *** indicates p < 0.001 compared to G1 group

It can be seen from the results in Table 14 that, compared with the control group, there were no significant difference in the number of hemolytic plaques of mice and there was no significant difference in the half hemolysis value of mice in serum (p > 0.05) in each dose group of active bacterial powder of *B. animalis* subsp. *lactis* HOM2120 strain (p > 0.05).

Table 15. The effect of bacterial powder of *B. animalis* subsp. *lactis* HOM2120 strain on the carbon clearance capacity and on the phagocytic rate and phagocytic index of mouse macrophages phagocytosing chicken red blood cells ( x̄±S)

| Group | Monocyte-Macrophage Function | | | |
|---|---|---|---|---|
| | Carbon clearance test | Experiment of macrophages phagocytosing chicken red blood cells | | |
| | Phagocytic index (a) | Phagocytic rate (%) | Square root of phagocytic rate Arcsine transform value | Phagocytic index |
| G1 (control group) | 5.89±0.60 | 12±6 | 0.35±0.10 | 0.19±0.09 |

(continued)

| Group | Carbon clearance test | Monocyte-Macrophage Function | | |
| | | Experiment of macrophages phagocytosing chicken red blood cells | | |
| | Phagocytic index (a) | Phagocytic rate (%) | Square root of phagocytic rate Arcsine transform value | Phagocytic index |
|---|---|---|---|---|
| G2 (HOM2120 low-dose group) | 6.59±0.61** | 21±8** | 0.47±0.09** | 0.32±0.11** |
| G3 (HOM2120 medium-dose group) | 6.57±0.52* | 20±5* | 0.46±0.06** | 0.31±0.07* |
| G4 (HOM2120 high-dose group) | 6.58±0.42** | 22±7** | 0.48±0.08*** | 0.35±0.13** |
| Note: * indicates $p < 0.05$ compared to G1 group; ** indicates $p < 0.01$ compared to G1 group; *** indicates $p < 0.001$ compared to G1 group | | | | |

It can be seen from the results in Table 15 that, compared with the control group, both the low-dose group and the high-dose group of active bacterial powder of the *B. animalis* subsp. *lactis* HOM2120 strain can significantly improve the carbon clearance capacity of mice ($p < 0.01$); the medium-dose group can significantly improve the carbon clearance capacity of mice ($p < 0.05$). Both the low-dose group and the high-dose group of active bacterial powder can significantly improve the phagocytic rate ($p < 0.01$) and phagocytic index ($p < 0.01$) of mouse macrophages phagocytosing chicken red blood cells; and the medium-dose group can significantly improve the phagocytic rate ($p < 0.05$) and phagocytic index ($p < 0.05$) of mouse macrophages phagocytosing chicken red blood cells. This indicated that active bacterial powder of *B. animalis* subsp. *lactis* HOM2120 strain significantly enhanced the function of mouse monocyte-macrophage.

Table 16. The effect of bacterial powder of *B. animalis* subsp. *lactis* HOM2120 strain on NK cell activity assay ($\overline{x}\pm S$)

| Group | Nk cell activity experiment | |
| | Nk cell activity (%) | Nk cell activity Square root arcsine transformation value |
|---|---|---|
| G1 (control group) | 27.1±5.6 | 0.55±0.06 |
| G2 (HOM2120 low-dose group) | 29.6±4.6 | 0.57±0.05 |
| G3 (HOM2120 medium-dose group) | 30.9±6.7 | 0.59±0.07 |
| G4 (HOM2120 high-dose group) | 30.9±3.5 | 0.59±0.04 |
| Note: Comparison between the test group and the negative control group, $p > 0.05$ | | |

As can be seen from the results in Table 16, there was no significant difference in NK cell activity of mice in each dose group of *B. animalis* subsp. *lactis* HOM2120 strain compared with the control group ($p > 0.05$).

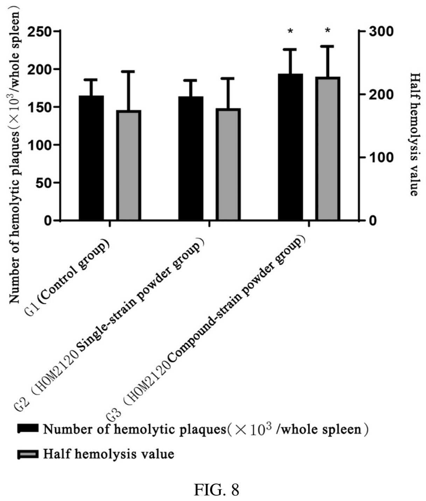

In summary, as shown in FIG. 8, compared with the control group, the active bacterial powder of *B. animalis* subsp. *lactis* HOM2120 strain can significantly enhance the cellular immune function and monocyte-macrophage function of mice, while having no effect on mouse body weight, thymus and spleen weight, humoral immune function and NK cell activity. In summary, the *B. animalis* subsp. *lactis* HOM2120 active bacterial powder has the function of enhancing immunity.

**Example 13. Animal Test 2 to Enhanced Immunity**

(1) Experimental animals and grouping: 144 female SPF-grade KM mice (18-20g) bred in Kunming by Beijing Huafukang Biotechnology Co., Ltd were selected. After animal adaptation and observation, they were randomly divided into four batches, with four groups per batch and 12 mice per group. The experimental groups were divided into a single-strain powder group (*B. animalis* subsp. *lactis* HOM2120, at a dose of $5 \times 10^{10}$ CFU/Kg BW), and the compound bacterial powder group (*B. animalis* subsp. *lactis* HOM2120 strain + *B. coagulans* HOM5301 strain (detailed information is recorded in the authorized Chinese patent CN112522134B, and the deposit number of this strain is CGMCC No. 20383) + *L. plantarum* HOM2217 strain, at a ratio of 1:1:1 (based on the number of live bacteria), at a dose of $1.5 \times 10^{11}$ CFU/Kg BW). They were administered by gavage daily with physiological saline as the solvent, which was administered orally once a day. Various indicators were measured after 32 days of continuous gavage.

Mice were administered with a volume of 10 mL/kg BW via gavage, while the control group was administered with a high dose of physiological saline solution containing the same mass of bacterial powder excipients via gavage. Each dose group was given maintenance feed. Experimental batch one underwent carbon clearance test; experimental batch two underwent organ/body weight ratio measurement and a delayed-type hypersensitivity experiment; experimental batch three underwent mouse peritoneal macrophage phagocytosis of chicken red blood cells experiments; and experimental batch four underwent experiment of ConA-induced transformation of mouse lymphocyte.

(2) Experimental Methods

Same as Example 12.

(3) Data Processing

Same as Example 12.

(4) Experimental Results

Table 17. The effects of the single-strain powder and the compound bacterial powder of *B. animalis* subsp. *lactis* HOM2120 strain on the body weight of mice ( $\bar{x}\pm S$ )

| Group | n | Initial Body Weight (g) | | Final Body Weight (g) | |
|---|---|---|---|---|---|
| | | Batch 1 | Batch 2 | Batch 1 | Batch 2 |
| G1(control group) | 12 | 22.5±1.7 | 22.5±1.2 | 35.5±2.8 | 35.1±2.5 |
| G2 (HOM2120 single-strain powder group) | 12 | 22.6±1.4 | 22.5±1.0 | 35.8±3.8 | 36.1±2.4 |
| G3 (HOM2120 compound bacterial powder group) | 12 | 22.5±1.2 | 22.4±0.9 | 35.4±3.1 | 34.9±2.6 |
| Group | n | Initial Body Weight (g) | | Final Body Weight (g) | |
| | | Batch 3 | Batch 4 | Batch 3 | Batch 4 |
| G1 (control group) | 12 | 22.4±1.1 | 22.4±0.7 | 36.2±2.8 | 35.3±3.2 |
| G2 (HOM2120 single-strain powder group) | 12 | 22.4±0.5 | 22.4±1.0 | 34.5±1.9 | 35.5±2.2 |
| G3 (HOM2120 compound bacterial powder group) | 12 | 22.4±0.9 | 22.4±0.8 | 36.0±2.1 | 36.6±3.6 |

Note: Comparison between each group and the negative control group, p > 0.05

It can be seen from the results in Table 17, compared with the control group, there was no significant difference in body weight before and after the experiment in the groups of single-strain powder and compound bacterial powder of *B. animalis* subsp. *lactis* HOM2120 strain (p > 0.05). This indicated that the single-strain powder and compound bacterial powder of the *B. animalis* subsp. *lactis* HOM2120 strain had no effect on the body weight of the mice.

Table 18. The effect of the single-strain powder and the compound bacterial powder of *B. animalis* subsp. *lactis* HOM2120 strain on organ/body weight ratio in mice ($\bar{x}\pm S$)

| Group | n | Spleen/Body (mg/g) | Thymus/Body (mg/g) |
|---|---|---|---|
| G1 (control group) | 12 | 4.86±0.60 | 4.02±0.87 |
| G2 (HOM2120 single-strain powder group) | 12 | 4.57±0.79 | 4.22±0.95 |
| G3 (HOM2120 compound bacterial powder group) | 12 | 4.66±0.62 | 4.12±0.72 |

Note: Comparison between the test group and the negative control group, compared, p > 0.05

It can be seen from the results in Table 18, compared with the control group, there was no significant difference in spleen/body weight and thymus/body weight of mice in the groups of single-strain powder and compound bacterial powder of *B. animalis* subsp. *lactis* HOM2120 strain (p > 0.05). This indicated that the single-strain powder and compound bacterial powder of the *B. animalis* subsp. *lactis* HOM2120 strain had no effect on the spleen and thymus weight of mice.

Table 19. The effect of the single-strain powder and the compound bacterial powder of *B. animalis* subsp. *lactis* HOM2120 strain on mice DTH induced by sheep red blood cells (SRBC) and on proliferation capacity of mouse spleen lymphocytes induced by ConA ( $\overline{x} \pm S$ )

| Group | n | Cell Immune Function Plantar Thickness Difference (mm) | Lymphocyte Proliferation Capacity (ΔOD Value) |
|---|---|---|---|
| G1 (control group) | 12 | 0.53±0.16 | 0.059±0.022 |
| G2 (HOM2120 single-strain powder group) | 12 | 0.69±0.18* | 0.084±0.022* |
| G3 (HOM2120 compound bacterial powder group) | 12 | 0.96±0.31*** | 0.101±0.058* |

Note: * indicates $p < 0.05$ compared to the control group; ** indicates $p < 0.01$ compared to the control group; *** indicates $p < 0.001$ compared to the control group.

It can be seen from the results in Table 19 that, compared with the control group, the single-strain powder group of *B. animalis* subsp. *lactis* HOM2120 strain can significantly improve the degree of mouse paw plantar thickening induced by sheep red blood cell ($p < 0.05$); and the compound bacterial powder group of *B. animalis* subsp. *lactis* HOM2120 strain can significantly improve the degree of mouse paw plantar thickening induced by sheep red blood cell ($p < 0.001$). The single-strain powder group and compound bacterial powder group of *B. animalis* subsp. *lactis* HOM2120 strain can significantly improve the proliferation capacity of mouse spleen lymphocytes induced by ConA ($p < 0.05$). This indicated that the single-strain powder and the compound bacterial powder of *B. animalis* subsp. *lactis* HOM2120 strain can significantly enhance the cellular immune function of mice. The compound bacterial powder was superior to the single-strain powder.

Table 20. The effect of the single-strain powder and the compound bacterial powder of *B. animalis* subsp. *lactis* HOM2120 strain on the number of antibody-producing cells and the half hemolysis value of serum ( $x \pm S$ )

| Group | Humoral Immune Function | |
|---|---|---|
| | Antibody-Production Cell Experiment | Serum Hemolysin Experiment |
| | The number of hemolytic plaques ($\times 10^3$/ whole spleen) | Half Hemolysis Value |
| G1 (control group) | 165±21 | 175±61 |
| G2 (HOM2120 single-strain powder group) | 164±21 | 178±47 |
| G3 (HOM2120 compound bacterial powder group) | 194±32* | 228±48* |

Note: * indicates $p < 0.05$ compared to G1 group; ** indicates $p < 0.01$ compared to G1 group; *** indicates $p < 0.001$ compared to G1 group

It can be seen from the results in Table 20, compared with the control group, there was no significant difference in the number of hemolytic plaques of mice ($p > 0.05$) and there was no significant difference in the half hemolysis value of mice in serum ($p > 0.05$) of single-strain powder group of *B. animalis* subsp. *lactis* HOM2120 strain. However, the compound bacterial powder of *B. animalis* subsp. *lactis* HOM2120 strain can significantly increase the number of hemolytic plaques in mice ($p < 0.05$), and can significantly increase the half hemolysis value (HC50) of mice ($p < 0.05$). This indicated that the compound bacterial powder of *B. animalis* subsp. *lactis* HOM2120 strain can significantly enhance of humoral immune function of mice.

Table 21. Effects of the single-strain powder and the compound bacterial powder of *B. animalis* subsp. *lactis* HOM2120 strain on the carbon clearance capacity and on the phagocytic rate and phagocytic index of mouse macrophages phagocytosing chicken red blood cells ( $\bar{x} \pm S$ )

| Group | Monocyte-Macrophage Function | | | |
| | Carbon clearance test | | Experiment of macrophages phagocytosing chicken red blood cells | |
| | Phagocytic index (a) | Phagocytic rate (%) | Square root of phagocytic rate Arcsine transform value | Phagocytic index |
| --- | --- | --- | --- | --- |
| G1 (control group) | 5.61±0.50 | 12±4 | 0.36±0.07 | 0.20±0.07 |
| G2 (HOM2120 single-strain powder group) | 6.70±0.96** | 17±4** | 0.43±0.06* | 0.28±0.07* |
| G3 (HOM2120 compound bacterial powder group) | 6.80±0.31*** | 20±5*** | 0.46±0.06*** | 0.35±0.11*** |

Note: * indicates $p < 0.05$ compared to G1 group; ** indicates $p < 0.01$ compared to G1 group; *** indicates $p < 0.001$ compared to G1 group

It can be seen from the results in Table 21 that, compared with the control group, the single-strain powder group of *B. animalis* subsp. *lactis* HOM2120 strain can significantly improve the carbon clearance capacity of mice ($p < 0.01$), and significantly improve the phagocytic rate ($p < 0.05$) and phagocytic index ($p < 0.05$) of chicken red blood cells phagocytosed by mouse macrophages. The compound bacterial powder group of *B. animalis* subsp. *lactis* HOM2120 strain can significantly improve the carbon clearance capacity of mice ($p < 0.001$), and significantly improve the phagocytic rate ($p < 0.001$) and phagocytic index ($p < 0.001$) of chicken red blood cells phagocytosed by mouse macrophages. This indicated that the active bacterial powder of the *B. animalis* subsp. *lactis* HOM2120 strain can significantly enhance the function of mouse monocyte-macrophage. The compound bacterial powder was superior to the single-strain powder.

Table 22. Effect of the single-strain powder and the compound bacterial powder of *B. animalis* subsp. *lactis* HOM2120 strain on NK cell activity assay ( $\bar{x} \pm S$ )

| Group | Nk cell activity experiment | |
| | Nk cell activity (%) | Nk cell activity Square root arcsine transformation value |
| --- | --- | --- |
| G1 (control group) | 28.2±5.4 | 0.56±0.06 |
| G2 (HOM2120 single-strain powder group) | 31.2±4.6 | 0.59±0.05 |
| G3 (HOM2120 compound bacterial powder group) | 29.0±5.7 | 0.57±0.07 |

Note: Comparison between the test group and the negative control group, $p > 0.05$

As can be seen from the results in Table 22, compared with the control group, there was no significant difference in NK cell activities of the mice in single-strain powder group and compound bacterial powder group of *B. animalis* subsp. *lactis* HOM2120 strain ($p > 0.05$).

In summary, compared with the control group, the single-strain powder of *B. animalis* subsp. *lactis* HOM2120 strain can significantly enhance the cellular immune function and monocyte-macrophage function of mice, while having no effect on mouse body weight, thymus and spleen weight, humoral immune function and NK cell activity. The compound bacterial powder of *B. animalis* subsp. *lactis* HOM2120 strain can significantly enhance the cellular immune function, humoral immune function and monocyte-macrophage function of mice, while having no effect on mouse body weight, thymus and spleen weight and NK cell activity. In summary, both the single-strain powder and compound bacterial powder of *B. animalis* subsp. *lactis* HOM2120 strain had the function of enhancing immunity, and the effect of the compound bacterial powder was better than that of the single-strain powder.

**Example 14. Animal Test of defecation Function**

(1) Experimental animals and grouping: a total of 48male SPF-grade KM mice (18-20g) bred in Kunming by Beijing Huafukang Biotechnology Co., Ltd were selected. After animal adaptation and observation, they were randomly divided into four groups, with 12 mice in each group for small intestinal animal test. The experimental groups were divided into a single-strain powder group (*B. animalis* subsp. *lactis* HOM2120, at a dose of $5 \times 10^{10}$ CFU/Kg BW), and the compound bacterial powder group (*B. animalis* subsp. *lactis* HOM2120 strain + *B. coagulans* HOM5301 strain + *L. plantarum* HOM2217 strain, at a ratio of 1:1:1 (based on the number of live bacteria), at a dose of $1.5 \times 10^{11}$ CFU/Kg BW).

(2) Experimental Methods

The mice were administered via gavage once daily for 15 consecutive days, with physiological saline as solvent. Mice in each group were fasted but allowed free access to water for 16 hours. The model control group and the experimental group were administered with compound diphenoxylate (5 mg/KgBW) by gavage, while the blank control group was administered with distilled water by gavage. 0.5 hours after administration of compound diphenoxylate, each sample dosage group was given ink (containing 5% activated carbon and 10% gum arabic) containing the corresponding test sample, and the blank control group and the model control group were administered with ink by gavage. After 25 minutes, the animals were immediately sacrificed by cervical dislocation. The intestinal lumen was opened, the mesentery was separated, and the intestinal segment from the pylorus (upper end) to the ileocecal junction (lower end) was cut. The segment was placed on the tray, and the small intestine was gently pulled into a straight line. The length of the intestinal segment was measured as "the total length of the small intestine", and the length from the pylorus to the front edge of the ink was measured as "ink propulsion length". The ink propulsion rate was calculated using the following formula:

Ink propulsion rate (%) = ink propulsion length (cm) / total length of the small intestine (cm) $\times$ 100%.

(3) Data Processing

Data conversion was required for the ink propulsion rate, X=Sin-1√p (p is the ink propulsion rate, expressed as a decimal). SPSS software was used for data processing. Independent sample T-test was used. The statistical results are divided into two parts, i.e., 1: Levene's test for homogeneity of variance; 2: t-test for equality of means. When the result of the homogeneous of variance test is Sig > 0.05, it is considered that the variances are homogeneous, so the Sig value in the first column of the T-test for equality of means is taken. When Sig ≤ 0.05, the statistical results between the two groups are considered to be different. When the result of the homogeneity of variance test is Sig ≤ 0.05, it is considered that the variances are not homogeneous, so the Sig value in the second column of the T-test for equality of means is taken. When Sig ≤ 0.05, it is considered that there is a difference in the statistical results between the two groups.

(4) Experimental Results

Table 23. The effect of single-strain powder and compound bacterial powder of *B. animalis* subsp. *lactis* HOM2120 on the ink propulsion rate ( $\bar{x} \pm S$ )

| Group | n | Ink propulsion rate (%) | Square root arcsine value of ink propulsion rate |
|---|---|---|---|
| G1 (blank control group) | 12 | 71.4±11.7 | 1.01±0.13 |
| G2 (Model Control) | 12 | 27.9±6.1### | 0.55±0.07### |
| G2 (HOM2120 single-strain powder group) | 12 | 34.2±8.3* | 0.62±0.09* |
| G3 (HOM2120 compound bacterial powder group) | 12 | 44.1±10.6*** | 0.73±0.11* |

Note: # indicates p < 0.05 compared to the blank control group; ## indicates p < 0.01 compared to the blank control group; ### indicates p < 0.01 compared to the blank control group.
*indicates p < 0.05 compared to the model control group; ** indicates p < 0.01 compared to the model control group; *** indicates p < 0.001 compared to the model control group.

It can be seen from the results in Table 23 that, after 15 days of oral administration with single-strain powder and compound bacterial powder of *B. animalis* subsp. *lactis* HOM2120 strain to mice, the constipation model of mice was induced using compound diphenoxylate. Compared with the blank control group, the model control group showed a decreased ink propulsion rate, with the P value showed a significant difference, indicating that the model was successfully established. Compared with the model control group, the ink promotion rate of single-strain powder and compound bacterial powder groups of *B. animalis* subsp. *lactis* HOM2120 strain was increased, and the p value showed a significant difference, the compound bacterial group was superior to the single-strain group. This indicates that the single-strain powder and compound bacterial powder of the *B. animalis* subsp. *lactis* HOM2120 strain has the potential of defecation Function.

The above description is only preferred embodiments of the present disclosure, and it should be noted that for those skilled in the art, several improvements and modifications can be made without departing from the principle of the present disclosure, and these improvements and modifications should also be considered within the protection scope of the present invention.

Although the embodiments of the present invention have been disclosed as above, they are not limited to the applications listed in the specification and the embodiments. They can be fully applied to various fields suitable for the present invention, and those skilled in the art can easily realize other modifications. Therefore, without departing from the general concept defined by the claims and the equivalent scope, the present invention is not limited to specific details and the details shown and described herein.

## Claims

1. A compound bacterial powder preparation, comprising a *Bifidobacterium animalis* (*B. animalis*) strain, a *Lactiplantibacillus plantarum* (*L. plantarum*) strain and a *Bacillus coagulans* (*B. coagulans*) strain, wherein the deposit number of the *B. animalis* strain is CGMCC No. 25681.

2. The compound bacterial powder preparation according to claim 1, wherein the compound bacterial powder preparation consists of *B. animalis* HOM2120 strain, *L. plantarum* HOM2217 strain and *B. coagulans* HOM5301 strain.

3. The compound bacterial powder preparation according to claim 1, wherein a ratio of the *B. animalis* strain, the *L. plantarum* strain and the *B. coagulans* strain is 1-3 : 1-3 : 1-3, based on the number of viable bacteria.

4. The compound bacterial powder preparation according to any one of claims 1 to 3, wherein the ratio of the *B. animalis* strain, the *L. plantarum* strain and the *B. coagulans* strain is 1:1:1, based on the number of viable bacteria.

5. The compound bacterial powder preparation according to any one of claims 1 to 4, wherein the deposit number of the *L. plantarum* strain is CGMCC No. 25683.

6. The compound bacterial powder preparation according to any one of claims 1 to 5 for use as a medicament for defecation.

7. The compound bacterial powder preparation according to any one of claims 1 to 5 for use in producing extracellular polysaccharides, lactic acid and short-chain fatty acids.

8. The compound bacterial powder preparation according to any one of claims 1 to 5 for use as a medicament for improving immunity and maintaining immune balance.

9. The compound bacterial powder preparation for use according to claim 8, wherein the compound bacterial powder preparation is used to produce cytokine, which is tumor necrosis factor-$\alpha$ (TNF-$\alpha$) and interleukin-6 (IL-6).

10. A method for preparing the compound bacterial powder preparation according to any one of claims 1 to 5, comprising: mixing the *B. animalis* strain, the *L. plantarum* strain and the *B. coagulans* strain, and compounding.

11. The method according to claim 10, wherein the ratio of the *B. animalis* strain, the *L. plantarum* strain and the *B. coagulans* strain is 1-3 : 1-3 : 1-3, based on the number of viable bacteria.

12. The method according to claim 10, wherein the ratio of the *B. animalis* strain, the *L. plantarum* strain and the *B. coagulans* strain is 1:1:1, based on the number of viable bacteria.

13. The method according to claim 10, wherein the compound bacterial powder preparation is in the form of a solution, powder, granules, tablet, or capsule.

14. The method according to claim 10, wherein the *L. plantarum* strain is isolated from healthy human breast milk, and the *B. animalis* strain is isolated from the feces of healthy infants.

15. The compound bacterial powder preparation according to any one of claims 1 to 5 for use as a medicament for losing weight.

FIG. 1A

FIG. 1B

FIG. 2

FIG. 3

FIG. 4

FIG. 5

FIG. 6

FIG. 7

FIG. 8

FIG. 9

FIG. 10

FIG. 11

## INTERNATIONAL SEARCH REPORT

| International application No. |
| --- |
| **PCT/CN2024/100577** |

| A. | CLASSIFICATION OF SUBJECT MATTER |
| --- | --- |

C12N1/20(2006.01)i; A61K35/747(2015.01)i; A61P1/10(2006.01)i

According to International Patent Classification (IPC) or to both national classification and IPC

| B. | FIELDS SEARCHED |
| --- | --- |

Minimum documentation searched (classification system followed by classification symbols)

IPC: C12N,A61K,A61P

Documentation searched other than minimum documentation to the extent that such documents are included in the fields searched

Electronic data base consulted during the international search (name of data base and, where practicable, search terms used)

DWPI, VEN, CNABS, CNTXT, CNKI, PubMed, GenBank, EBI, STN, WPABSC, WPABS, ENTXT, ENTXTC, Wanfang Database, ISI web of Knowledge: 双歧杆菌, 动物双歧杆菌, 植物乳杆菌, 植物乳植杆菌, 凝结芽孢杆菌, 益生菌, 免疫, TNF-α, IL-6, 肥胖, 胆固醇, Bifidobacterium animalis, Lactiplantibacillus plantarum, Bacillus coagulans, HOM2120, HOM2217, HOM5301, CGMCC NO.25681, CGMCC NO.25683

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| PY | CN 117721032 A (KELI CO., LTD.) 19 March 2024 (2024-03-19) claims 1-10 | 1-15 |
| PY | CN 117327607 A (KELI CO., LTD.) 02 January 2024 (2024-01-02) claims 1-10 | 1-15 |
| PY | CN 117327603 A (KELI CO., LTD.) 02 January 2024 (2024-01-02) claims 1-12 | 1-15 |
| PY | CN 117327622 A (KELI CO., LTD.) 02 January 2024 (2024-01-02) claims 1-11 | 1-15 |
| PY | CN 117327623 A (KELI CO., LTD.) 02 January 2024 (2024-01-02) claims 1-15 | 1-15 |
| Y | CN 112522134 A (KELI CO., LTD.) 19 March 2021 (2021-03-19) claims 1-10 | 1-15 |

| ✓ Further documents are listed in the continuation of Box C. | ✓ See patent family annex. |
| --- | --- |

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **24 July 2024** | **29 August 2024** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
| --- |
| **PCT/CN2024/100577** |

**C.        DOCUMENTS CONSIDERED TO BE RELEVANT**

| Category* | Citation of document, with indication, where appropriate, of the relevant passages | Relevant to claim No. |
| --- | --- | --- |
| A | CN 104017751 A (QINGDAO JIUHEYISHENG BIOLOGICAL TECHNOLOGY CO., LTD.) 03 September 2014 (2014-09-03) claims 1-8 | 1-15 |
| A | CN 111567809 A (OXLOO CO., LTD.) 25 August 2020 (2020-08-25) claims 1-10 | 1-15 |
| A | US 2020268812 A1 (EWELINA SOSNOWSKA-TUREK et al.) 27 August 2020 (2020-08-27) claims 1-2, and description, embodiments | 1-15 |
| A | FIJAN, S. "Probiotics and Their Antimicrobial Effect" *microorganisms,* Vol. 11, 19 February 2023 (2023-02-19), article 528, pages 1-4 | 1-15 |

**INTERNATIONAL SEARCH REPORT**

International application No.

**PCT/CN2024/100577**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT
### Information on patent family members

International application No.

**PCT/CN2024/100577**

| Patent document cited in search report | | | Publication date (day/month/year) | Patent family member(s) | | | Publication date (day/month/year) |
|---|---|---|---|---|---|---|---|
| CN | 117721032 | A | 19 March 2024 | None | | | |
| CN | 117327607 | A | 02 January 2024 | None | | | |
| CN | 117327603 | A | 02 January 2024 | None | | | |
| CN | 117327622 | A | 02 January 2024 | None | | | |
| CN | 117327623 | A | 02 January 2024 | None | | | |
| CN | 112522134 | A | 19 March 2021 | None | | | |
| CN | 104017751 | A | 03 September 2014 | None | | | |
| CN | 111567809 | A | 25 August 2020 | None | | | |
| US | 2020268812 | A1 | 27 August 2020 | US | 11045507 | B2 | 29 June 2021 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202310744483
- CN 202311294783
- CN 202311294650
- CN 112522134 B

**Non-patent literature cited in the description**

- The B. animalis HOM2120 strain of the present invention was deposited in the China General Microbiological Culture Collection Center (CGMCC). *Institute of Microbiology, Chinese Academy of Sciences*, 09 September 2022 (3)

- Bergey's Manual of Systematic Bacteriology. *International Journal of Systematic and Evolutionary Microbiology*